# EUROPEAN PATENT APPLICATION

(11) **EP 4 265 600 A1**
(43) Date of publication of application: **25.10.2023**
(21) Application number: 21905633.0
(22) Date of filing: 10.12.2021
(51) Int. Cl.: C07D 265/06, C07D 263/44, C07D 273/00, C07D 317/36, C08G 69/10, C08G 69/04

(54) **PROCESS FOR PREPARING CARBOXYL CYCLIC ACID ANHYDRIDE**

(30) Priority: 16.12.2020 CN 202011486166
(71) Applicant: Peking University, Beijing 100871 (CN)
(72) Inventor: LU, Hua, Beijing 100871 (CN); TIAN, Ziyou, Beijing 100871 (CN)
(74) Representative: Cabinet Becker et Associés
(86) International application number: PCT/CN2021/137111
(87) International publication number: WO 2022/127704

(57) **Abstract**

Disclosed are a method for preparing a carboxyl cyclic acid anhydride and a prepared product thereof. A method for preparing a compound of formula (II), comprising the steps of reacting a compound of formula (I) with a cyclization reagent to produce the compound of formula (II) and an acid, and using an epoxy compound as an acid scavenger. The present invention relates to a new process for synthesizing a carboxyl cyclic acid anhydride, which is low in cost, and has low requirements for reagents, places and equipment required for experiments, mild conditions, a wide application range of substrates and a high tolerance of functional groups. The synthesis route can recycle some solvents and other high value-added by-products, with less waste liquid discharge and easy amplification. It also has extremely high industrial and academic value, and can greatly promote the rapid mass production and application of products such as polyamino acid, polyhydroxy acid (polyester), and polymercaptic acid (polysulfide).

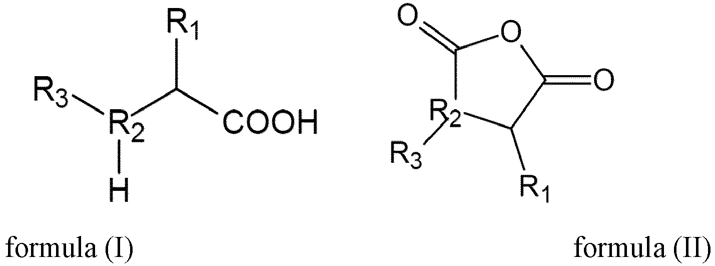

## Description

### FIELD OF THE INVENTION

The invention relates to a preparation method of a compound, in particular to a preparation method of carboxyanhydrides.

### BACKGROUND TO THE INVENTION

Polyamino acids (also known as polypeptide polymers, synthetic polypeptides, etc.) are important bio-based polymers, which can be used as both drugs and pharmaceutical preparations, and have important applications in high-end biomaterials, cosmetics, asymmetric catalysis and many other high value-added fields, with huge market potential. For example, polylysine can be used in cell culture, antibacterial materials and gene transfection materials. Polyglutamic acid and aspartic acid can be used in pharmaceutical preparations, carriers and medical materials. Many nano-drugs based on polyglutamic acid and aspartic acid have entered the clinical trial stage in the United States, Japan and China. Polyvaline and polyleucine are used as catalysts for asymmetric Julia-Colonna epoxidation in industry. Polysarcosine can be used in pharmaceutical preparations and carriers. Glatiramer acetate is a kind of polyamino acid, L-alanine-L-glutamic acid-L-lysine-L-tyrosine polypeptide polymer acetate (prepared by polymerization of four NCAs), and its sales in 2012 reached 4 billion dollars, ranking among the top 20 best-selling drugs in the world all the year round.

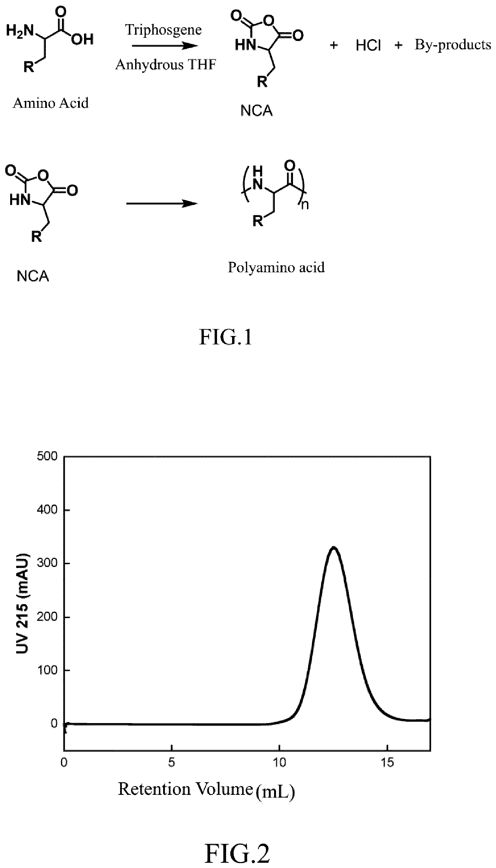

Polyamino acids are mainly prepared by ring-opening polymerization of amino acid N-carboxyanhydrides (hereinafter referred to as NCA); NCAis usually prepared by cyclization synthesis of corresponding amino acids (or their derivatives) (Figure 1). It has been more than 100 years since Leuch discovered NCA in 1906. The cyclizing agents used successively include thionyl chloride, phosgene, triphosgene, PCl3, PCl5, etc. At present, triphosgene is the most common one (Kricheldorf, H. R., Polypeptides and 100 years of chemistry of alpha-amino acid N-carboxyanhydrides. Angew. Chem., Int. Ed. 2006, 45(35), 5752-5784). However, no matter what cyclizing agent is used, the NCA synthesis in industry and laboratory needs strict anhydrous conditions, including the use of expensive anhydrous solvents such as anhydrous tetrahydrofuran (hereinafter referred to as THF), the protection with dry nitrogen during the reaction, and heating to 50-60 degrees in a closed reagent bottle. The reaction produces a large amount of waste liquid containing high concentration of hydrochloric acid, which corrodes the equipment and increases the difficulty of waste liquid treatment. What is more serious is that the post-treatment of NCA is extremely complicated and difficult to operate, and the risk of no grain harvest can be faced with a little carelessness.

There is a need in the field for a preparation method of carboxyanhydrides, especially amino acid N-carboxyanhydrides, which overcomes the defects of the existing methods.

### SUMMARY OF THE INVENTION

In the long-term work, the inventor found that the traditional synthesis of amino acid N-carboxyanhydrides (NCA) requires repeated recrystallization in a glove box with anhydrous solvent. For NCA which cannot be crystallized, it needs to be separated by silica gel column in glove box with anhydrous solvent (Kramer, J. R.; Deming, T. J., General Method for Purification of alpha-Amino acid-N-carboxyanhydrides Using Flash Chromatography. Biomacromolecules 2010, 11(12), 3668-3672). Because NCA polymerization requires high monomer purity, it is often necessary to repeat the above separation steps for some NCAs. In addition, the traditional synthesis methods usually need to protect pendant functional groups such as hydroxyl and sulfhydryl, and after polymerization, it is necessary to add deprotection steps to release these functional groups, which increases the synthesis steps and costs. Due to the above restrictions, the industrial production cost of NCA is high; this further leads to the high cost of downstream product polyamino acids, which limits its large-scale production and numerous applications. If NCA without side group protection can be purified and separated with high yield outside the glove box by using common solvents without nitrogen protection, it will greatly reduce the synthesis difficulty and cost of polyamino acids, thereby promoting the larger-scale and wider use of polyamino acids. In view of these defects in the prior art, the inventor provides a method for preparing carboxyanhydrides, especially amino acid N-carboxyanhydrides, which uses epoxy compounds as hydrochloric acid removal reagents in the NCA synthesis process. More importantly, the new method makes some monomers which are difficult to synthesize or cannot be obtained become cheap and easy to obtain, thereby further promoting the simple and controllable synthesis of some new polyamino acids and protein conjugates thereof. It has shown a wide application prospect in the fields of protein drugs and biomaterials.

In addition, the method of the present invention can be used not only for cyclizing amino acids to produce amino acid N-carboxyanhydrides (NCA), but also for cyclizing amino acid derivatives to produce carboxyanhydrides, as shown in the following formula 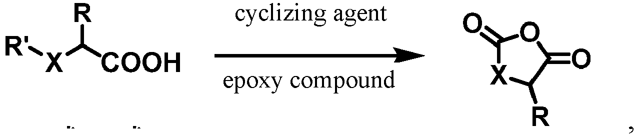 where X=NH, O or S, and the corresponding products are NCA, OCA and SCA.

R' can be H, Boc or Cbz.

In one aspect, the present invention provides a preparation method, which comprises the steps of reacting an amino acid, a hydroxy acid, a sulfhydryl acid or a derivative thereof in which some groups (such as amino group, sulfhydryl group, hydroxyl group or carboxyl group, such as α-amino group or ε-amino group) are protected with a cyclizing agent to prodcue N-, O-or S-carboxyanhydride and an acid, and using an epoxy compound as an acid removal agent, provided that the amino acid, hydroxy acid or sulfhydryl acid or the derivative thereof can form the corresponding carboxyanhydride.

In one aspect, the present invention provides a method for preparing a compound of formula (II), which comprises the steps of reacting a compound of formula (I) with a cyclizing agent to generate a compound of formula (II) and an acid, and using an epoxy compound as an acid removal agent. 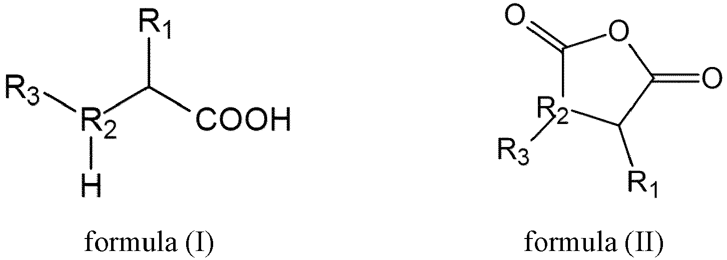 wherein:
R₁ is -R₄-R₅-R₆, wherein R₄ is H, optionally substituted C₁₋₆ linear or branched alkyl (for example, C₁₋₆ linear or branched alkyl substituted by hydroxyl or sulfhydryl), optionally substituted C₁₋₆ alkoxy, carboxyl, amino, carbonylamino (-CO-NH₂), guanidyl, optionally substituted phenyl (for example, substituted by hydroxyl or alkyl), optionally substituted benzyl (for example, substituted by hydroxyl or alkyl), indolyl, imidazolyl, guanidyl or carbonylalkoxy;
R₅ is absent, oxy (-O-), seleno (-Se-), thio (-S-), carbonyl (-CO-), ester group (carbonyloxy, -COO-), ester group imino (-COO-NH-) (or oxycarbonylimino), imino, amino, carbonylamino(-CO-NH₂), carbonylimino(-CO-NH-), benzyl ester group (BnCOO-), optionally substituted phenyl (e.g., substituted by hydroxyl), optionally substituted benzyl(e.g., substituted by hydroxyl), indolyl, imidazolyl, guanidyl, carbonylalkoxy, or carboxyl;
R₆ is absent, H, optionally substituted C₁₋₆ linear or branched alkyl (for example, C₁₋₆ linear or branched alkyl substituted by hydroxyl or sulfhydryl), optionally substituted C₁₋₆ alkoxy, hydroxyl, carboxyl, sulfhydryl, amino, carbonylamino, guanidyl, optionally substituted phenyl (for example, substituted by hydroxyl), amino protecting group, hydroxyl protecting group or carboxyl protecting group, optionally substituted benzyl (for example, substituted by hydroxyl), optionally substituted phenylalkyl, optionally substituted benzylalkyl, indolyl, imidazolyl, guanidyl, carbonylalkoxy, benzyl ester group, benzyliminocarbonylalkyl or -[O(CH₂)ₘ₁]ₘ₂-R₇,
wherein R₇ is H, optionally substituted C₁₋₆ linear or branched alkyl (for example, C₁₋₆ linear or branched alkyl substituted by hydroxyl or sulfhydryl), optionally substituted C₁₋₆ alkoxy, carbonylalkyl or iminoalkyl; each of m1 and m2 is independently an integer from 1 to 6;
R₂ is N, O or S, or R₁ and R₂ are taken together with the carbon atom to which they are attached to form a 3- to 7-membered ring, the 3- to 7-membered ring is optionally substituted with halogen, sulfhydryl or hydroxyl, the 3- to 7-membered ring is, for example, indolyl, imidazolyl, pyrrolidinyl, sulfhydrylpyrrolidinyl or hydroxypyrrolidinyl; when R₂ is O or S, H connected to R₂ is optionally substituted by hydroxyl protecting group or sulfhydryl protecting group;
R₃ is absent, H, halogen, optionally substituted C₁₋₆ linear or branched alkyl (for example, C₁₋₆ linear or branched alkyl substituted by hydroxyl or sulfhydryl), optionally substituted C₂₋₆ linear or branched alkenyl or alkynyl, C₁₋₆ alkoxy, cycloalkyl, aryl or heterocyclyl, wherein one or more hydrogen atoms are optionally substituted by halogen, oxygen or nitrogen, or Boc or Cbz.

In one embodiment, the epoxy compound as an acid removal agent has the structure of formula (III): 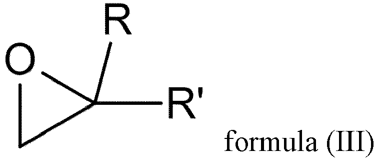 wherein: R and R' are independently H, halogen, optionally substituted C₁₋₆ linear or branched alkyl, optionally substituted C₂₋₆ linear or branched alkenyl or alkynyl, alkoxy or cycloalkyl; wherein one or more hydrogen atoms are optionally substituted by halogen, C₁₋₆ linear or branched alkyl, oxygen or nitrogen; or, one of R and R' together with the two carbon atoms in the epoxy group to form a 5-to 7-membered ring.

In one embodiment, the epoxy compound is selected from one or more of the following: ethylene oxide, propylene oxide, 1,2-butylene oxide, dimethyl ethylene oxide, cyclohexene oxide and halogen-substituted derivatives thereof, such as epichlorohydrin.

In one embodiment, the cyclizing agent is selected from one or more of the following: phosgene, diphosgene, triphosgene, phosphorus trichloride and phosphorus pentachloride.

In one embodiment, R₁ is H, optionally substituted C₁₋₆ linear or branched alkyl, optionally substituted aryl or BnNHCOCH₂CH₂SeCH₂CH₂; preferably, the optionally substituted C₁₋₆ linear or branched alkyl is linear or branched C₁₋₆ alkyl, C₁₋₆ alkylhydroxyl, C₁₋₆ alkylsulfhydryl, C₁₋₆ alkylcarboxyl, C₁₋₆ alkylamino, C₁₋₆ alkylcarbonylamino, C₁₋₆ alkylaryl, C₁₋₆ alkylheterocyclyl, C₁₋₆ alkylguanidyl, C₁₋₆ alkylthioC₁₋₆ alkyl, C₁₋₆ alkyloxyC₁₋₆ alkyl. Preferably, aryl or heterocyclyl is optionally substituted, for example, by hydroxyl or sulfhydryl. Preferably, amino, hydroxyl or carboxyl is protected.

In one embodiment, R₁ is H, CH₃, CH(CH₃)₂, CH₂CH(CH₃)₂, CH(CH₃)CH₂CH₃, CH₂-C₆H₅, methylindolyl (such as 3-methylindolyl), CH₂-C₆H₄-OH, CH₂-COOH, CH₂-CONH₂, (CH₂)₂-COOH, (CH₂)₄-NH₂, (CH₂)₂-CONH₂, (CH₂)₂-S-CH₃, CH₂-OH, CH(CH₃)-OH, CH₂-SH, methylimidazolyl, BnCO₂NH(CH₂)₄, BnCO₂(CH₂)₂, CH₂-CH₂-CH₂-CN₃H₄, BnNHCOCH₂CH₂SeCH₂CH₂, CH₃O(CH₂CH₂O)₃COCH₂CH₂, SHC(CH₃)₂, C₆H₅, or C(CH₃)₃OCH₃ or trifluoroacetyliminobutyl. These groups can also be optionally substituted, such as substituted by halogen, alkyl, halogen, hydroxyl, sulfhydryl, carboxyl or aryl. In one embodiment, the amino group, hydroxyl group or carboxyl group on R₁ can be protected. The selection and protection methods of these groups are known to those skilled in the art.

In one embodiment, R₂ is N or O. In one embodiment, R₃ is absent, H or methyl. In one embodiment, R₁ and R₂ are taken together with the carbon atoms to which they are attached to form pyrrolidinyl or hydroxypyrrolidinyl, optionally with the imino group therein is protected. Those skilled in the art should understand that amino, imino, carboxyl, hydroxyl or sulfhydryl and the like can be protected. The selection and protection methods of these groups are known to those skilled in the art. For example, N-tert-butoxycarbonyl glycine, N-tert-butoxycarbonyl sarcosine or L-N-tert-butoxycarbonyl proline can be used.

In one embodiment, the compound of formula (I) is an amino acid or a derivative thereof, such as an amino acid protected by Boc or Cbz, which is selected from the following group: glycine, alanine, valine, leucine, isoleucine, methionine, proline, tryptophan, serine, tyrosine, cysteine, phenylalanine, asparagine, glutamine, threonine, aspartic acid, glutamic acid, lysine, arginine, histidine, ε-N-benzyloxycarbonyl lysine, benzyl glutamate, sarcosine, hydroxyproline, (S)-2-amino-4-((3-(benzylamino)-3-oxypropyl)seleno)butanoic acid, γ-(2-(2-(2-(2-methoxyethoxy)ethoxy)glutamic acid, penicillamine, L-phenyllactic acid, L-mandelic acid, or O-tert-butyl-serine or ε-N-trifluoroacetyl L-lysine. The compound of formula (II) can be the corresponding carboxyanhydride, such as N- or O-carboxyanhydride. In one embodiment, some amino acids, such as lysine or glutamic acid with amino groups or hydroxyl groups on the side chains that need to be protected, such as ε-N-trifluoroacetyl L-lysine, ε-N-benzyloxycarbonyl lysine, benzyl glutamate or γ-(2-(2-(2-(2-methoxyethoxy) ethoxy)glutamic acid.

In one embodiment, the method is carried out under atmospheric condition.

In another embodiment, the method is carried out at room temperature or without heating.

In one embodiment, the preparation method also includes the step of crystallization and/or column chromatography.

In another aspect, the present invention provides a method of forming a copolymer, which may include a step of forming a compound of formula (II) and a step of forming a copolymer. Preferably, R₁ is a carboxyl-containing group, such as COOHCH₂CH₂. For example, the compound of formula (I) is glutamic acid, and the compound of formula (II) is L-glutamic acid N-carboxyanhydride. Preferably, the copolymer is poly L-glutamic acid N-carboxyanhydride).

The copolymer can be represented by the following formula: 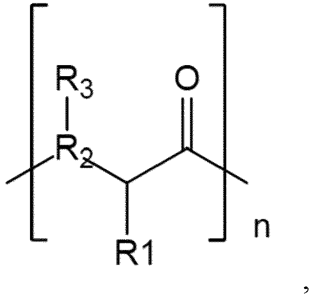 N is an integer greater than or equal to 2, for example 10, 20, 30, 40, 50, 60, 70, 80 or 90. For example, the copolymer is 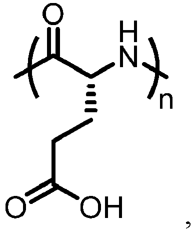 wherein n is 20.

In another aspect, the present invention provides a method for preparing a compound of formula (V), which comprises the steps of reacting a compound of formula (IV) with a cyclizing agent to generate a compound of formula (V) and an acid, and using an epoxy compound as an acid removal agent, 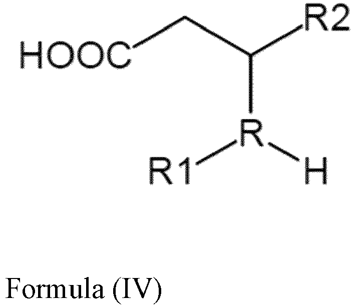 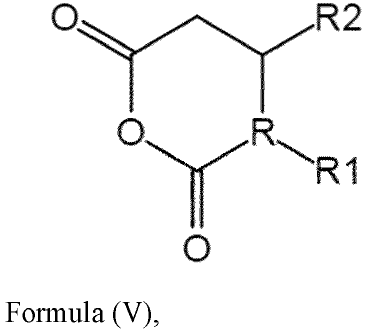 wherein R is N, O or S; R₁ is absent, H, halogen, optionally substituted C₁₋₆ linear or branched alkyl, optionally substituted C₂₋₆ linear or branched alkenyl or alkynyl, C₁₋₆ alkoxy, cycloalkyl, aryl or heterocyclyl, in which one or more hydrogen atoms are optionally substituted by halogen, oxygen or nitrogen, or Boc or Cbz; R₂ is H, optionally substituted C₁₋₆ linear or branched alkyl (for example, C₁₋₆ linear or branched alkyl substituted by hydroxyl or sulfhydryl) or optionally substituted C₁₋₆ alkoxy or carbonyloxyalkyl, such as methyl ester group, ethyl ester group and propyl ester group; when R is O or S, H connected to R is optionally substituted by hydroxyl protecting group or sulfhydryl protecting group.

In one embodiment, the epoxy compound as an acid removal agent has the structure of formula (III): 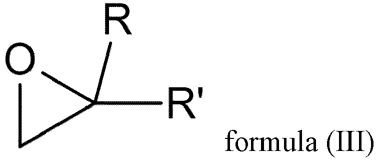 wherein: R and R' are independently H, halogen, optionally substituted C₁₋₆ linear or branched alkyl, optionally substituted C₂₋₆ linear or branched alkenyl or alkynyl, alkoxy or cycloalkyl; wherein one or more hydrogen atoms are optionally substituted by halogen, C₁₋₆ linear or branched alkyl, oxygen or nitrogen; alternatively, one of R and R' together with two carbon atoms in the epoxy group to form a 5- to 7-membered ring.

In one embodiment, the epoxy compound is selected from one or more of the following: ethylene oxide, propylene oxide, 1,2-butylene oxide, dimethyl ethylene oxide, cyclohexene oxide and halogen-substituted derivatives thereof, such as epichlorohydrin.

In one embodiment, the cyclizing agent is selected from one or more of the following: phosgene, diphosgene, triphosgene, phosphorus trichloride and phosphorus pentachloride.

In one embodiment, the method is carried out under atmospheric condition, and/or the method is carried out at room temperature or without heating.

In one embodiment, the preparation method also includes the step of crystallization and/or column chromatography.

In one embodiment, the compound of formula (IV) is 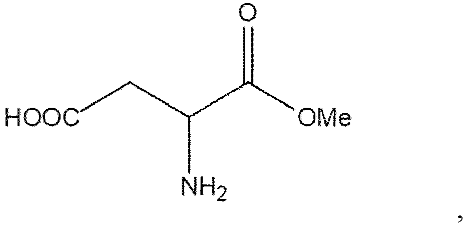 β-alanine, 3-amino-3-(4-methylphenyl)propionic acid, or 3-amino-3-(4-chlorophenyl)propionic acid, and the compound of formula (V) is 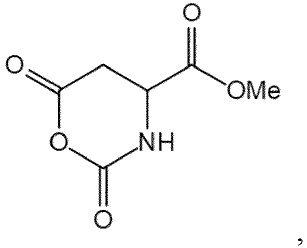 β-alanine N-carboxyanhydride, 3-amino-3-(4-chlorophenyl)propionic acid N-carboxyanhydride or 3-amino-3-(4-methylphenyl)propionic acid N-carboxyanhydride.

In another aspect, the present invention provides a method of forming a copolymer, which may include a step of forming a compound of formula (V) and a step of forming a copolymer.

In one embodiment, the copolymer is 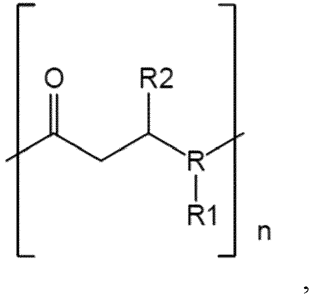 N is an integer greater than or equal to 2, such as 10, 20, 30, 40, 50, 60, 70, 80 or 90. For example, the copolymer is polyamino acid N-carboxyanhydride. For example, the copolymer is 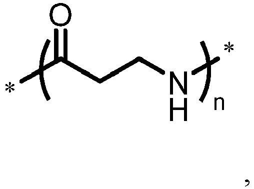 wherein n is 50.

The above polymerization step may includes the step of adding N,N-dimethyl formyl and benzylamine initiators to the starting materials of reaction, for example, mixing the N,N-dimethyl formyl and benzylamine initiators with the compound of formula (II) or (V).

The above compounds of formula (I) and formula (IV) can include all the starting amino acids or amino acid derivatives in the examples. The above compounds of formula (II) and formula (V) can include all the reaction products in the examples, that is, N- or O-carboxyanhydride, especially amino acid-N-carboxyanhydride.

In another aspect, the invention provides a compound prepared according to the preparation method of the invention.

In one embodiment, the compound of formula (II) has one of the following structures:

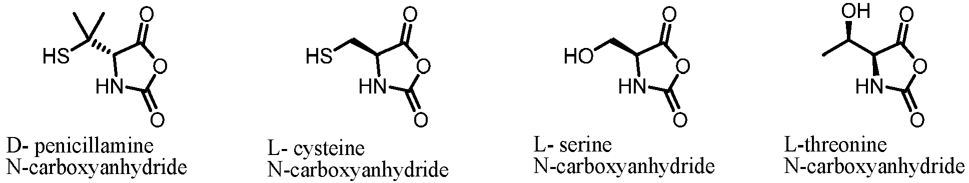

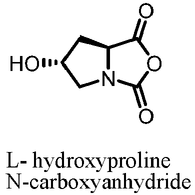

In another aspect, the present invention provides the use of the amino acid N-carboxy anhydrides prepared herein in the manufacturing of polyamino acids. For the carboxyanhydride, for example, the amino acid N-carboxyanhydride prepared herein, an epoxy compound can be used as an acid removal agent.

The present invention also provides a preparation method of the carboxyanhydride of an amino acid or a derivative thereof, which includes the steps of reacting the amino acid or the derivative thereof with a cyclizing agent to generate the carboxyanhydride of the amino acid or the derivative thereof and an acid, and using an epoxy compound as the acid removal agent. In one embodiment, the amino acid or the derivative thereof is selected from the group consisting of glycine, alanine, valine, leucine, isoleucine, methionine, proline, tryptophan, serine, tyrosine, cysteine, phenylalanine, asparagine, glutamine, threonine, aspartic acid, glutamate, lysine, arginine, histidine, ε-N-benzyloxycarbonyl lysine, benzyl glutamate, sarcosine, hydroxyproline, (S)-2-amino-4-((3-(benzylamino)-3-oxypropyl)seleno)butyric acid, penicillamine, L-phenyllactic acid, L-mandelic acid, or O-tert-butyl-serine, L-aspartic acid-1-methyl ester or 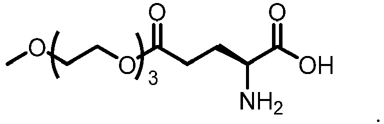 Derivatives can also be the corresponding Boc or Cbz protected amino acids of the above 20 natural amino acids. The method of the present invention is suitable for amino acids, hydroxy acids or sulfhydryl acids, and their derivatives where certain groups, such as amino groups, hydroxyl groups or carboxyl groups, such as α-amino groups or ε-amino groups are protected, as long as they can form corresponding carboxyanhydrides, such as N-, S- or O-carboxyanhydrides. Those skilled in the art can easily determine whether the groups need to be protected and the groups and methods for protecting these groups.

The present invention can also include the method or step of forming a polyamino acid from the above amino acid N-carboxyanhydride.

In the present invention, the epoxy compound can be added before the cyclizing agent is added. The method of the present invention can also include the step of removing the protecting group, such as Boc or Cbz.

The present invention also provides a method for synthesizing a polymer, which comprises preparing a compound of formula (II) and/or a compound of formula (V) by using the above method, and polymerizing one or more identical or different compounds of formula (II) and/or one or more identical or different compounds of formula (V) to form a polymer. Preferably, the polymer is a polyamino acid. For example, the degree of polymerization is 4, 5, 6, 7, 8, 9, 10, 20, 30, 40, 50, 60, 70, 80 or 90.

The present invention also provides a preparation method of L-alanine-L-glutamic acid-L-lysine -L-tyrosine polypeptide polymer, which comprises reacting L-alanine, L-glutamic acid derivative, L-lysine derivative or L-tyrosine with cyclizing agent to generate the corresponding amino acid N-carboxyanhydride and an acid, wherein an epoxy compound is used as acid removal agent in the preparation process of one or more corresponding amino acid N-carboxyanhydride. Then the corresponding amino acid N-carboxyanhydride is polymerized to form L-alanine-L-glutamic acid-L-lysine-L-tyrosine polypeptide polymer.Preferably, the cyclizing agent is selected from one or more of the following: phosgene, diphosgene, triphosgene, phosphorus trichloride and phosphorus pentachloride. Preferably, the epoxy compound is the epoxy compound defined above. Preferably, the above derivatives are amino acids with protected carboxyl or amino groups. For example, the derivative of L- glutamic acid is benzyl L- glutamate, and/or the derivative of L-lysine is N-ε-trifluoroacetyl -L- lysine.

Advantages of the method of the present invention include:
1. Compared with other acid removal agents, the reaction between epoxy compound and hydrochloric acid in THF is extremely fast, and it can be completed almost instantaneously at room temperature. Not only can the acid be completely removed, but also chloride ions can be removed.
2. The price of epoxy compound is lower than that of pinene and limonene. So the cost is low.
3. The product 1-chloro-2-propanol or 2-chloro-1-propanol produced by the epoxy compound and hydrochloric acid can be easily separated and purified and sold as chemical materials. The above characteristics determine that this method is not only cheap, but also has nearly zero emission, less environmental pollution, and can also produce some high value-added by-products.
4. In both the synthesis itself and the subsequent recrystallization/column chromatography, anhydrous solvent and glove box can be completely abandoned, and high-purity NCA monomer can be obtained in high yield by using cheap analytical pure ordinary solvents in the whole process under the conventional experimental environment.
5. Without heating and nitrogen protection, the reaction rate is faster than that of ordinary methods.
6. Universality and functional group compatibility are good. For some monomers that cannot be obtained by ordinary methods or are extremely difficult to synthesize, they can be successfully prepared and clearly characterized by using the present method.

### BRIEF DESCRIPTION OF THE FIGURES

Figure 1: Existing NCA synthesis method and the polymerization to prepare polyamino acids.
Fig. 2: SEC characterization of poly L-glutamic acid-N-carboxyanhydride.

### DETAILED DESCRIPTION OF THE INVENTION

As used herein, "amino acid" may refer to a compound having the structure H₂N-R^{x}-COOH, wherein R^{x} is an organic group, and wherein NH₂ can optionally be combined with R^{x} (e.g., as in the case of proline). Amino acid derivatives refer to compounds obtained by substituting the atoms of amino acids. Amino acids include any known amino acids, including but not limited to α amino acids, β amino acids, γ amino acids, δ amino acids, etc. In some embodiments, the term can refer to α or β amino acids. As used herein, amino acids can be 20 natural amino acids, such as glycine, alanine, valine, leucine, isoleucine, methionine, proline, tryptophan, serine, tyrosine, cysteine, phenylalanine, asparagine, glutamine, threonine, aspartic acid, glutamic acid, lysine, arginine or histidine. Or, amino acid derivatives refer to compounds obtained by substituting the atoms of amino acids, such as ε-N-benzyloxycarbonyl lysine, benzyl glutamate, sarcosine, hydroxyproline, (S)-2-amino-4-((3-(benzylamino)-3-oxopropyl)seleno)butyric acid, γ-(2-(2-(2-(2-methoxyethoxy)ethoxy)glutamic acid, penicillamine, phenyllactic acid, mandelic acid, or O-tert-butyl-serine, or amino acids protected by protecting groups such as Boc or Cbz, such as various naturally occurring amino acids protected by Boc or Cbz. The protection of protecting groups can be the protection for α-amino groups, hydroxyl groups, sulfhydryl groups and carboxyl groups, or the protection for thecorrespond groups in side chains, or both. The above amino acids can be L-amino acids or D-amino acids. In one embodiment, R₁ is H, CH₃, CH(CH₃)₂, CH₂CH(CH₃)₂, CH(CH₃)CH₂CH₃, CH₂-C₆H₅, indolyl (C₈NH₆), CH₂-C₆H₄-OH, CH₂-COOH, CH₂-CONH₂, (CH₂)₂-COOH, (CH₂)₄-NH₂, (CH₂)₂-CONH₂, (CH₂)₂-S-CH₃, CH₂-OH, CH(CH₃)-OH, CH₂-SH, -C₃H₆, imidazolyl or guanidyl.

As used herein, an "epoxy compound" is a kind of compound with a three-membered cyclic ether structure. For example, an epoxy compound can have the structure shown in Formula (III): 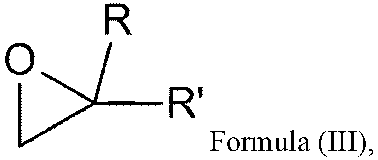 wherein: R and R' are independently H, halogen, optionally substituted C₁₋₆ linear or branched alkyl, optionally substituted C₂₋₆ linear or branched alkenyl or alkynyl, alkoxy or cycloalkyl; wherein one or more hydrogen atoms are optionally substituted by halogen, C₁₋₆ linear or branched alkyl, oxygen or nitrogen; or one of R and R' together with two carbon atoms in the epoxy group to form a 5- to 7-membered ring. Specifically, the epoxy compound can be ethylene oxide, propylene oxide, 1,2-butylene oxide, dimethyl ethylene oxide, cyclohexyloxirane and halogen-substituted derivatives thereof, such as chlorinated, brominated or fluorinated ethylene oxide, propylene oxide, 1,2-butylene oxide, dimethyl ethylene oxide or cyclohexyloxirane.

As used herein, "cyclizing agent" refers to an agent that catalyzes the cyclization of amino acids or derivatives thereof (such as Formula I shown herein) to provide carboxyanhydrides. For example, the cyclizing agent can be selected from phosgene, diphosgene, triphosgene, phosphorus trichloride and phosphorus pentachloride.

As used herein, "halogen" refers to a fluorine, chlorine, bromine or iodine atom. In some embodiments, halogen can be a chlorine atom.

As used herein, "alkyl" refers to a linear or branched chain saturated hydrocarbon with 1 to 30 carbon atoms, which can be optionally substituted, as further described herein, allowing various degrees of substitution. As used herein, examples of "alkyl" include, but are not limited to, methyl, ethyl, n-propyl, isopropyl, isobutyl, n-butyl, sec-butyl, tert-butyl, isopentyl, n-pentyl, neopentyl, n-hexyl and 2-ethylhexyl. In some cases, one or more carbon atoms in alkyl group can be replaced by heteroatoms, and are called heteroalkyl group. Non-limiting examples include alkoxy, which refers to a group in which carbon atom(s) in alkyl group is(are) replaced by oxygen. Alkyl can be C₁₋₆ alkyl. "C₁₋₆ alkyl" means alkyl with 1 to 6 carbon atoms, and includes, for example, but not limited to methyl, ethyl, n-propyl, isopropyl, isobutyl, n-butyl, sec-butyl, tert-butyl, isopentyl, n-pentyl, neopentyl and n-hexyl. "C₁₋₆ alkoxy" represents a group in which one or more carbon atoms in C₁₋₆ alkyl are replaced by oxygen, such as methoxy, ethoxy, n-butoxy, tert-butoxy, pentoxy or hexyloxy. "Alkyl" or "alkoxy" can also be substituted by halogen, alkyl, hydroxyl, sulfhydryl, carboxyl, aryl, etc. Alkoxy can be C₁₋₆ alkoxy.

As used herein, "alkenyl" refers to a linear or branched chain nonaromatic hydrocarbon with 2 to 30 carbon atoms and one or more carbon-carbon double bonds, which can be optionally substituted, as further described herein, allowing various degrees of substitution. As used herein, examples of "alkenyl" include, but are not limited to, vinyl, 2-propenyl, 2-butenyl and 3-butenyl. In some cases, one or more carbon atoms in alkenyl or alkenylene group can be substituted by heteroatoms (such as nitrogen, oxygen or sulfur when feasible) and are called "heteroalkenyl group". "Alkenyl group" can also be substituted by halogen, alkyl, hydroxyl, sulfhydryl, carboxyl, aryl, etc.

As used herein, aryl can be phenyl, biphenyl or naphthyl, and can be mono-, di- or tri-substituted, for example, by halogen, hydroxyl, alkyl, methyl, sulfhydryl, carboxyl, etc. Substituents derived from aryl groups can be substituted like aryl groups, such as aralkyl, aryloxy, arylthio or arylacyl groups.

As used herein, heteroaryl can be an aromatic mono- or bi-cyclic heterocyclyl containing 5 to 7 or 8 to 12 ring atoms respectively, wherein 1 to 2 ring atoms are sulfur atoms or oxygen atoms or 1 to 4 ring atoms are nitrogen atoms, which can be understood as thienyl, benzo[b]thienyl, furyl, pyranyl, pyranyl, benzofuryl, pyrrolyl, imidazolyl, pyrazolyl, pyridyl, pyrimidinyl, pyridazinyl, indazolyl, isoindolyl, indolyl, purinyl, quinolinyl, isoquinolinyl, 2,3-diazanaphthyl, 1,5-diazanaphthyl, quinoxalinyl, quinazolinyl, cinnolinyl, pteridinyl, oxazolyl, isoxazolyl, thiazolyl or isothiazolyl. Heteroaryl can also be substituted by halogen, alkyl, hydroxyl, sulfhydryl, carboxyl, aryl, etc.

As used herein, "substitution" or "substituted" refers to the substitution of one or more hydrogen atoms of a specified moiety with one or more specified substituents, and unless otherwise specified, various degrees of substitution are allowed, provided that the substitution results in a stable or chemically feasible compound. "Optionally substituted" means that the group may or may not be substituted. Substitution may be substituted by halogen, alkyl, hydroxyl, sulfhydryl, carboxyl and aryl.

### The method of the present invention

The inventor found that high concentration hydrochloric acid will be produced in the synthesis of NCA, and if it encounters water in the process of synthesis and purification, hydrochloric acid will catalyze the decomposition or polymerization of NCA, which will lead to the failure of synthesis. Based on this reason, the existing literatures and patents report methods of adding acid removal agents such as organic amine (such as triethylamine) (Gkikas, M.; Avery, R. K.; Olsen, B. D., Thermoresponsive and Mechanical Properties of Poly(L-proline) Gels. Biomacromolecules 2016, 17(2), 399-406), pinene (Fabrice Comille, B. S. Y.; Jean-Luc Copier, A.; Jean-Pierre Senet, B.; Yves Robin, V. L. P. PROCESS FOR THE PREPARATION OF N-CARBOXYANHYDRIDES. US 6,479,665 B2, 2002), limonene (Smeets, N. M. B.; van der Weide, P. L. J.; Meuldijk, J.; Vekemans, J. A. J. M.; Hulshof, L. A., A Scalable Synthesis ofl-Leucine-N-carboxyanhydride. Org. Process Res. Dev. 2005, 9(6), 757-763) in the synthesis process. However, the inventor found that because amine is a kind of alkali, excessive amount will lead to NCA polymerization, and insufficient amount will not be enough to completely eliminate the side effects of hydrochloric acid, so it is not easy to control the equivalent amount in practical operation, and the triethylammonium chloride byproduct produced by triethylamine and hydrochloric acid has good solubility in water and organic solvents and is not easy to remove; moreover, triethylamine can not remove nucleophilic chloride ions, which can still induce NCA decomposition, so the effect is not ideal. (Biomacromolecules 2011, 12, 6, 2396-2406; J. Org. Chem. 1965, 30, 4, 1158-1161). Moreover, pinene reacts slowly with hydrochloric acid in tetrahydrofuran (the most common solvent for NCA synthesis). (J. Am. Chem. Soc. 1941, 63, 3, 860-862), and hydrochloric acid can not be removed in time and effectively in the synthesis process, so anhydrous solvent and nitrogen protection are still needed. At the same time, pinene and limonene are expensive, which increases the cost of industrial production. (Smeets, N. M. B.; van der Weide, P. L. J.; Meuldijk, J.; Vekemans, J. A. J. M.; Hulshof, L. A., A Scalable Synthesis ofl-Leucine-N-carboxyanhydride. Org. Process Res. Dev. 2005, 9(6), 757-763).

After long-term exploration, the inventor found that using epoxy compound such as propylene oxide (PO) as hydrochloric acid removal reagent in NCA synthesis process overcome the shortcomings of the prior art. The reaction between epoxy compound such as PO and hydrochloric acid in THF is very fast, and it can be completed almost instantaneously at room temperature, which can not only completely remove acid but also remove chloride ions. The price of epoxy compounds such as PO is cheaper than that of pinene and limonene, so the cost is low (the price of PO is RMB 169 yuan/L; the price of pinene is 771 yuan/kg; the price of limonene is 1129 yuan/kg; Data source: cheapest price of each chemical in Peking University reagent management platform). In addition, the product 1-chloro-2-propanol or 2-chloro-1propanol produced by PO and hydrochloric acid can be easily separated and purified and sold as chemical materials. The above characteristics determine that this method is not only cheap, but also has nearly zero-emission, with little environmental pollution, and can also produce some by-products with high added value.

Therefore, the present invention especially provides a preparation method of amino acid N-carboxyanhydride, which includes the steps of reacting amino acid with cyclizing agent to generate amino acid N-carboxyanhydride and an acid, and using epoxy compound as acid removal agent. The amino acid can be any kind of amino acid, for example, those as defined above. In this context, the acid can be hydrogen halide, such as hydrogen chloride or hydrogen bromide. The cyclizing agent can be those as defined above, including ethylene oxide, propylene oxide, 1,2-butylene oxide, dimethyl ethylene oxide, cyclohexene oxide and halogen-substituted derivatives thereof. When this method is used, all the advantages mentioned above can be achieved. The method can include adding amino acids or derivatives thereof into a container, adding a solvent, such as analytically pure tetrahydrofuran, adding an epoxy compound, such as propylene oxide, stirring uniformly (for example, under magnetic stirring), adding a cyclizing agent, such as triphosgene under atmospheric conditions, and then stirring (for example, sealing or with a gas guide plug slightly opened).

The invention also provides a method for preparing amino acid N-carboxyanhydride, which includes the above steps. The method can also include the step of reacting the obtained amino acid N-carboxyanhydride with N,N-dimethylformamide and benzylamine initiator. Preferably, the side chain of the amino acid contains carboxyl group, for example, R1 group is an optionally substituted C₁₋₆ linear or branched alkylcarboxyl group. For example, the amino acid is glutamic acid.

For example, the method includes adding tetrahydrofuran and epichlorohydrin to L-glutamic acid, stirring uniformly, adding triphosgene under atmospheric condition, reacting at room temperature, drying, and crystallizing to obtain the final product L-glutamic acid N-carboxyanhydride. The method can also include adding N,N-dimethylformamide and benzylamine initiators to L-glutamic acid N-carboxyanhydride, and reacting at room temperature to obtain the final product poly L-glutamic acid N-carboxyanhydride with a polymerization degree N of 20, as shown in the examples. Compared with the traditional methods, the present method for preparing polyamino acid N-carboxyanhydrides can be used to prepare polyamino acid N-carboxyanhydrides with higher yield.

### Examples

The following examples show some illustrative embodiments of the compounds, compositions and methods disclosed herein. These examples should not be regarded as limiting in any way. These examples should not be regarded as expressing any preferred embodiments or indicating any direction for further research. The experimental methods used in the following examples are all conventional methods unless otherwise specified, and the materials, reagents, etc. used are commercially available.

### Example 1: Synthesis of benzyl L-glutamate N-carboxyanhydride

Benzyl L-glutamate (5.0 g, 21.1 mmol, 1 equivalent) was put into a thick-walled pressure-resistant bottle, and 60 mL of analytically pure tetrahydrofuran, propylene oxide (15.3 mL, 211 mmol, 10 equivalent) and triphosgene (3.184 g, 10.5 mmol, 0.5 equivalent) were added. The solution was stirred at room temperature for 1.5 hours, washed with water, dried and subjected to diffusion crystallization to obtain the final product benzyl L-glutamate N-carboxyanhydride (5.159 g, yield 93%).

The NMR and carbon spectra of benzyl L-glutamate N-carboxyanhydride are as follows:
**¹H NMR**(400 MHz, CDCl₃) δ 7.42 - 7.31(m, 5H), 6.70(s, 1H), 5.13(s, 2H), 4.38(ddd, *J* = 6.7, 5.4, 1.0 Hz, 1H), 2.59(t, *J* = 6.9 Hz, 1H), 2.32 - 2.21(m, 1H), 2.18 - 2.06(m, 1H).
**¹³C NMR**(101 MHz, CDCl₃) δ 172.5, 169.6, 152.2, 135.3, 128.8, 128.7, 128.4, 67.2, 57.0, 29.8, 26.9.

### Example 2: Synthesis of glycine N-carboxyanhydride

N-tert-butoxycarbonyl glycine (1.0 g, 5.7 mmol, 1 equivalent) was added to a 100 mL round-bottomed flask, followed by addition of analytically pure acetonitrile (20 mL), propylene oxide (4 mL, 57.1 mmol, 10 equivalent) and triphosgene (861 mg, 2.9 mmol, 0.5 equivalent). Reaction was carried out for 1.5 hours, and the solution was subjected to spin-drying, crystallization to provide the final product glycine N-carboxyanhydride (357 mg, yield 62%).

The NMR and carbon spectra of glycine N-carboxyanhydride are as follows:
**¹H NMR**(400 MHz, DMSO-*d*₆) δ 8.83(s, 1H), 4.18(s, 2H).
**¹³C NMR**(101 MHz, DMSO-*d*₆) δ 169.4, 153.0, 46.3.

### Example 3: ε-N-benzyloxycarbonyl L-lysine N-carboxyanhydride

ε-N-benzyloxycarbonyl L-lysine (1.0 g, 3.6 mmol, 1 equivalent) was added to a thick-walled pressure-resistant bottle, and analytically pure tetrahydrofuran (15 mL), propylene oxide (2.5 mL, 36 mmol, 10 equivalent) and triphosgene (534 mg, 1.8 mmol, 0.5 equivalent) were added. The reaction was stirred at room temperature for 1.5 hours, washed with water, dried and crystallized to obtain the final product ε-N-benzyloxycarbonyl L-lysine N-carboxyanhydride (929 mg, yield 85%).

The NMR and carbon spectra of ε- N-benzyloxycarbonyl L-lysine N-carboxyanhydride are as follows:
**¹H** NMR(400 MHz, DMSO-*d*₆) δ 9.08(s, 1H), 7.40 - 7.28(m, 5H), 7.26(t, J= 6.1 Hz, 1H), 5.00(s, 2H), 4.43(dd, *J* = 7.4, 5.1 Hz, 1H), 2.99(dt, *J* = 6.1, 6.1 Hz, 2H), 1.80 - 1.58(m, 1H), 1.48 - 1.22(m, 4H).
**¹³C NMR**(101 MHz, DMSO-*d*₆) δ 171.7, 156.1, 152.0, 137.3, 128.4, 127.8, 127.8, 124.2, 65.2, 57.0, 30.7, 28.8, 21.6.

### Example 4: Synthesis of L-alanine N-carboxyanhydride

L-alanine (1.0 g, 11.2 mmol, 1 equivalent) was added into a thick-walled pressure-resistant bottle, 30 mL of analytically pure tetrahydrofuran, propylene oxide (8.0 mL, 112.2 mmol, 10 equivalent), and triphosgene (1.674 g, 5.6 mmol, 0.5 equivalent) were added. The reaction was stirred at room temperature for 19.5 hours, washed with water, dried and crystallized to obtain the final product L-alanine N-carboxyanhydride (821 mg, yield 64%).
The NMR and carbon spectra of L-alanine N-carboxyanhydride are as follows: **¹H NMR**(400 MHz, CDCl₃) δ 6.76(br, 1H), 4.42(qd, *J* = 7.0, 1.0 Hz, 1H), 1.56(d, *J* = 7.0 Hz, 3H).
**¹³C NMR**(101 MHz, CDCl₃) δ 170.3, 152.6, 53.5, 17.8.

### Example 5: Synthesis of L-tyrosine N-carboxyanhydride

L-tyrosine (1.0 g, 6.1 mmol, 1 equivalent) was added to a thick-walled pressure-resistant bottle, and analytically pure tetrahydrofuran (15 mL), propylene oxide (4.3 mL, 61 mmol, 10 equivalent) and triphosgene (897 mg, 3.0 mmol, 0.5 equivalent) were added. The reaction was stirred at room temperature for 7 hours, washed with water, dried and crystallized to obtain the final product, L-tyrosine N-carboxyanhydride (961 mg, yield 83%).
The NMR and carbon spectra of L-tyrosine N-carboxyanhydride are as follows: **¹H NMR**(400 MHz, DMSO-*d*₆) δ 9.34(br, 1H), 9.03(br, 1H), 6.97(d, *J* = 8.4 Hz, 2H), 6.69(d, *J* = 8.4 Hz, 2H), 4.70(td, *J* = 5.0, 1.0 Hz, 1H), 2.93(dd, *J* = 14.5, 5.0 Hz, 1H), 2.89(dd, *J* = 14.5, 5.0 Hz, 1H).
**¹³C NMR**(101 MHz, DMSO-*d*₆) δ 170.9, 156.5, 151.7, 130.7, 124.6, 115.2, 58.5, 35.5.

### Example 6: Synthesis of sarcosine N-carboxyanhydride

N-tert-butoxycarbonyl sarcosine (1.0 g, 5.3 mmol, 1 equivalent) was added to a 100 mL round-bottomed flask, then analytically pure acetonitrile (20 mL) was added, propylene oxide (3.7 mL, 53 mmol, 10 equivalent) was added under ice-water bath and then triphosgene (793 mg, 2.7 mmol, 0.5 equivalent) was added. The reaction was carried out for 1.5 hours, washed with water, dried and crystallized to obtain the final product, sarcosine N-carboxyanhydride (381 mg, yield 63%).
The NMR and carbon spectra of sarcosine N-carboxyanhydride are as follows: **¹H NMR**(400 MHz, CDCl₃) δ 4.13(s, 2H), 3.03(s, 3H).
**¹³C NMR**(101 MHz, CDCl₃) δ 165.5, 152.4, 51.0, 30.4.

### Example 7: Synthesis of L-hydroxyproline N-carboxyanhydride

L-N-tert-butoxycarbonyl hydroxyproline (5.0 g, 21.6 mmol, 1 equivalent) was added to a 250 mL round-bottomed flask, and analytically pure acetonitrile (50 mL) was added, propylene oxide (15.1 mL, 216 mmol, 10 equivalent) was added under ice-water bath and then triphosgene (3.222 g, 10.8 mmol, 0.5 equivalent) was added. The reaction was carried out for 1.5 hours, washed with water, dried and passed through silica gel column to obtain the final product, L-hydroxyproline N-carboxyanhydride (2.696 g, purity: mass fraction 95%, yield 75%, containing 5%EA).
The NMR and carbon spectra of L-hydroxyproline N-carboxyanhydride are as follows:
**¹H NMR**(400 MHz, DMSO-*d*₆) δ 5.31(br, 1H), 4.72(dd, *J* = 10.8, 6.8 Hz, 1H), 4.54(ddd, *J* = 4.8, 4.8, 1.5 Hz, 1H), 3.71(dd, *J* = 11.2, 4.8 Hz, 1H), 3.06(dd, *J* = 11.2,1.5 Hz, 1H), 2.23(ddd, *J* = 12.2, 10.8, 4.8 Hz, 1H), 1.95(dd, *J* = 12.2, 6.8 Hz, 1H).
**¹³C NMR**(101 MHz, DMSO-*d*₆) δ 170.3, 154.7, 72.6, 62.3, 55.0, 35.5.

### Example 8: L-methionine N-carboxyanhydride

L-methionine (1.0 g, 6.7 mmol, 1 equivalent) was added to a thick-walled pressure-resistant bottle, then analytically pure tetrahydrofuran (20 mL), propylene oxide (4.7 mL, 67 mmol, 10 equivalent) and triphosgene (1.002 g, 3.4 mmol, 0.5 equivalent) were added. The reaction was stirred at room temperature for 2 hours, washed with water, dried and passed through silica gel column to obtain the final product, L-methionine N-carboxyanhydride (700 mg, yield 60%).
The NMR and carbon spectra of L-methionine N-carboxyanhydride are as follows: **¹H NMR**(400 MHz, CDCl₃) δ 7.06(br, 1H), 4.51(ddd, *J* = 7.4, 5.0, 1.1 Hz, 1H), 2.67(t, *J* = 6.6 Hz, 2H), 2.25(dtd, *J* = 14.6, 6.6, 5.0 Hz, 1H), 2.17 - 2.01(m, 1H), 2.09(s, 3H).
**¹³C NMR**(101 MHz, CDCl₃) δ 169.9, 152.9, 56.7, 30.2, 29.8, 15.2.

### Example 9: L-tryptophan N-carboxyanhydride

L-tryptophan (1.0 g, 4.9 mmol, 1 equivalent) was added to a thick-walled pressure-resistant bottle, and 15 mL of analytically pure tetrahydrofuran, propylene oxide (3.4 mL, 49 mmol, 10 equivalent) and triphosgene (749 mg, 2.5 mmol, 0.5 equivalent) were added. The reaction was stirred at room temperature for 1.5 hours, washed with water, dried and crystallized to obtain the final product, L-tryptophan N-carboxyanhydride (1.020 g, yield 90%).
The NMR and carbon spectra of L-tryptophan N-carboxyanhydride are as follows: **¹H NMR**(400 MHz, DMSO-*d*₆) δ 11.00(d, *J* = 2.5 Hz, 1H), 9.09(s, 1H), 7.55(d, *J* = 7.9 Hz, 1H), 7.36(d, *J* = 8.1 Hz, 1H), 7.15(d, *J* = 2.5 Hz, 1H), 7.13 - 7.05(m, 1H), 7.05 - 6.96(m, 1H), 4.78(t, *J* = 5.0 Hz, 1H), 3.22(dd, *J* = 15.0, 5.0 Hz, 1H), 3.14(dd, *J* = 15.0, 5.0 Hz, 1H).
**¹³C NMR**(101 MHz, DMSO-*d*₆) δ 171.3, 151.9, 136.0, 127.2, 124.5, 121.1, 118.6, 118.5, 111.5, 107.1, 58.3, 26.5.

### Example 10: L-proline N-carboxyanhydride

L-N-tert-butoxycarbonyl proline (1.0 g, 4.7 mmol, 1 equivalent) was added to a 100 mL round-bottomed flask, analytically pure acetonitrile (15 mL) was added, propylene oxide (3.7 mL, 47 mmol, 10 equivalent) was added under ice-water bath and then triphosgene (700 mg, 2.3 mmol, 0.5 equivalent) was added. The reaction was carried out for 2.5 hours, washed with water, dried and passed through silica gel column to obtain the final product L-proline N-carboxyanhydride (470 mg, yield 72%).
The NMR and carbon spectra of L-proline N-carboxyanhydride are as follows:
**¹H** NMR(400 MHz, CDCl₃) δ 4.32(dd, *J* = 9.1, 7.4 Hz, 1H), 3.73(dt, *J* = 11.3, 7.4 Hz, 1H), 3.30(ddd, *J* = 11.3, 8.5, 4.6 Hz, 1H), 2.28(dtd, *J* = 12.4, 7.4, 3.7 Hz, 1H), 2.24 - 2.14(m, 1H), 2.09(dtd, *J* = 14.3, 9.1, 7.4, 4.6 Hz, 1H), 1.92(dq, *J* = 12.4, 9.1 Hz, 1H).
**¹³C NMR**(101 MHz, CDCl₃) δ 168.9, 154.9, 63.1, 46.5, 27.6, 26.9.

### Example 11: L-phenylalanine N-carboxyanhydride

L-phenylalanine (1.0 g, 6.1 mmol, 1 equivalent) was added to a thick-walled pressure-resistant bottle, and 15 mL of analytically pure tetrahydrofuran, propylene oxide (4.3 mL, 60 mmol, 10 equivalent) and triphosgene (897 mg, 4.0 mmol, 0.5 equivalent) were added. The reaction was stirred at room temperature for 7 hours, washed with water, dried and crystallized to obtain the final product, L-phenylalanine N-carboxyanhydride (961 mg, yield 83%).
The NMR and carbon spectra of L-phenylalanine N-carboxyanhydride are as follows:
**¹H NMR**(400 MHz, CDCl₃) δ 7.38 - 7.27(m, 3H), 7.17(dd, *J* = 7.7, 1.8 Hz, 2H), 6.48(br, 1H), 4.53(ddd, *J* = 7.8, 4.3, 1.0 Hz, 1H), 3.24(dd, *J* = 14.2, 4.3 Hz, 1H), 3.01(dd, *J* = 14.2, 7.8 Hz, 1H).
**¹³C NMR**(101 MHz, CDCl₃) δ 168.9, 152.1, 134.0, 129.3, 128.1, 59.0, 37.9.

### Example 12: Synthesis of L-serine N-carboxyanhydride

L-serine (1.0 g, 9.5 mmol, 1 equivalent) was added into a thick-walled pressure-resistant bottle, then analytically pure dioxane (30 mL), propylene oxide (6.7 mL, 95.2 mmol, 10 equivalent), and triphosgene (1.415 g, 4.8 mmol, 0.5 equivalent) were added. The reaction was stirred at room temperature for 4 hours, passed through silica gel column, crystallized to obtain the final product, L-serine N-carboxyanhydride (890 mg, yield 71%).
The NMR and carbon spectra of L-serine N-carboxyanhydride are as follows:
**¹H NMR**(400 MHz, THF-*d*₈) δ 7.89(brs, 1H), 4.69(brs, 1H), 4.35 - 4.32(m, 1H), 3 . 81 (dd, *J* = 11.6, 3 .4 Hz, 1 H), 3 .71 (dd, *J* = 11. 6, 2. 7 Hz, 1 H) .
**¹³C NMR**(101 MHz, THF-ds) δ 170.6, 153.6, 61.6, 61.5.

### Example 13: Synthesis of L-threonine N-carboxyanhydride

L-threonine (2.0 g, 8.4 mmol, 1 equivalent) was added into a thick-walled pressure-resistant bottle, 30 mL of analytically pure tetrahydrofuran, propylene oxide (12 mL, 95.2 mmol, 10 equivalent), and triphosgene (2.5 g, 4.8 mmol, 0.5 equivalent) were added. The reaction was stirred at room temperature for 12 hours, washed with water, dried and crystallized to obtain the final product, L-threonine N-carboxyanhydride (1.186 g, yield 48%).
The NMR and carbon spectra of L-threonine N-carboxyanhydride are as follows:
**¹H NMR**(400 MHz, DMSO-*d*₆) δ 9.05(brs, 1H), 5.17(brs, 1H), 4.32(dd, *J* = 2.3, 1.1 Hz, 1H), 3.98(qd, *J* = 6.6, 2.3 Hz, 1H), 1.14(d, *J* = 6.6 Hz, 3H).
**¹³C NMR**(101 MHz, DMSO-*d*₆) δ 170.5, 152.7, 65.4, 63.7, 19.9.

### Example 14: Synthesis of γ-(2-(2-(2-(2-methoxyethoxy)ethoxy) L-glutamic acid N-carboxyanhydride

γ-(2-(2-(2-(2-methoxyethoxy)ethoxy) L-glutamic acid amino acid (2.82 g, 9.6 mmol, 1 equivalent) was added to a thick-walled pressure-resistant bottle, 50 mL of analytically pure tetrahydrofuran, propylene oxide (6.7 mL, 96.1 mmol, 10 equivalent), and triphosgene (1.475 g, 4.8 mmol, 0.5 equivalent) were added. The reaction was stirred at room temperature for 2 hours, passed through silica gel column to obtain the final product γ-(2-(2-(2-(2-methoxyethoxy)ethoxy) L-glutamic acid N-carboxyanhydride (2.206 mg, yield 72%).
The NMR and carbon spectra of γ-(2-(2-(2-(2-methoxyethoxy)ethoxy) L-glutamic acid N-carboxyanhydride are as follows:
**¹H NMR**(400 MHz, DMSO-*d*₆) δ 9.09(br, 1H), 4.46(ddd, *J* = 7.9, 5.4,1.2 Hz, 1H), 4.20 - 4.07(m, 2H), 3.60(dd, *J* = 5.3, 4.2 Hz, 2H), 3.57 - 3.47(m, 5H), 3.46 - 3.39(m, 2H), 3.24(s, 3H), 2.47(t, *J* = 7.6 Hz, 2H), 2.10 - 1.96(m, 1H), 1.99 - 1.83(m, 1H).
**¹³C NMR**(101 MHz, DMSO-*d*₆) δ 171.8, 171.4, 151.9, 71.3, 69.8, 69.7, 69.6, 68.2, 63.5, 58.1, 56.2, 29.1, 26.5.

### Example 15: (S)-2-amino-4-((3-(benzylamino)-3-oxypropyl)seleno)butyric acid N-carboxyanhydride

(S)-2-amino-4-((3-(benzylamino)-3-oxypropyl)seleno)butyric acid hydrochloride (1.0 g, 2.7 mmol, 1 equivalent) was added to a thick-walled pressure-resistant bottle, and 15 mL of analytically pure tetrahydrofuran, propylene oxide (2.0 mL, 27 mmol, 10 equivalent) and triphosgene (397 mg, 1.3 mmol, 0.5 equivalent) were added. The reaction was stirred at room temperature for 1.5 hours, washed with water, dried and passed through silica gel column to obtain the final product, (*S*)-2-amino-4-((3-(benzylamino)-3-oxypropyl)seleno)butyric acid N-carboxyanhydride (570 mg, yield 58%).
The NMR and carbon spectra of (*S*)-2-amino-4-((3-(benzylamino)-3-oxypropyl)seleno)butyric acid N-carboxyanhydride are as follows:
**¹H NMR**(400 MHz, DMSO-*d*₆) δ 9.11(br, 1H), 8.40(t, *J* = 5.9 Hz, 1H), 7.36 - 7.19(m, 5H), 4.52(ddd, *J* = 7.8, 5.2, 1.2 Hz, 1H), 4.28(d, *J* = 5.9 Hz, 2H), 2.75(t, *J* = 7.3 Hz, 2H), 2.69 - 2.58(m, 2H), 2.55(t, *J* = 7.3 Hz, 2H), 2.12 - 2.00(m, 2H).
**¹³C NMR**(101 MHz, DMSO-*d*₆) δ 171.4, 170.8, 152.0, 139.4, 128.3, 127.2, 126.7, 56.9, 42.1, 36.5, 31.9, 18.4, 18.3.

### Example 16: L-cysteine N-carboxyanhydride

L-cysteine (10.0 g, 82.5 mmol, 1 equivalent) was added to a thick-walled pressure-resistant bottle, and analytically pure tetrahydrofuran (120 mL), propylene oxide (23 mL, 330.2 mmol, 4 equivalents) and triphosgene (12.313 g, 41.3 mmol, 0.5 equivalents) were added. The reaction was stirred at room temperature for 5 hours, spin-dried and crystallized to obtain the final product L-cysteine N-carboxyanhydride (9.443 g, yield 78%).
The NMR and carbon spectra of L-cysteine N-carboxyanhydride are as follows (adding a little DMSO- *d*₆ to THF- ds):
**¹H NMR**(400 MHz, THF-ds) δ 13.36 - 12.30(br, 1H), 8.27(br, 1H), 4.35(ddd, *J* = 8.5, 3.9, 1.4 Hz, 1H), 3.69(dd, *J* = 11.2, 8.5 Hz, 1H), 3.47(dd, *J* = 11.2, 3.9 Hz, 1H).
**¹³C NMR**(101 MHz, THF-ds) δ 173.4, 173.2, 56.8, 32.7.

### Example 17: L-phenyllactic acid O-carboxyanhydride

L-phenyllactic acid (1.0 g, 6.0 mmol, 1 equivalent) was added to a thick-walled pressure-resistant bottle, and analytical pure tetrahydrofuran (15 mL), propylene oxide (4.2 mL, 60 mmol, 10 equivalent) and triphosgene (893 mg, 3.0 mmol, 0.5 equivalent) were added. The reaction was stirred at room temperature for 24 hours, washed with water, dried and crystallized to obtain the final product, L-phenyllactic acid O-carboxyanhydride (750 mg, yield 65%).
The NMR and carbon spectra of L-phenyl lactic acid O-carboxyanhydride are as follows:
**¹H NMR**(400 MHz, CDCl₃) δ 7.41 - 7.29(m, 3H), 7.26 - 7.19(m, 2H), 5.30(t, *J* = 4.9 Hz, 1H), 3.38(dd, *J* = 14.9, 4.9 Hz, 1H), 3.25(dd, *J* = 14.9, 4.9 Hz, 1H).
**¹³C NMR**(101 MHz, CDCl₃) δ 166.5, 147.9, 131.6, 129.8, 129.3, 128.5, 80.0, 36.5.

### Example 18: Synthesis of L-mandelic acid O-carboxyanhydride

L-mandelic acid (1.0 g, 6.6 mmol, 1 equivalent) was added to a thick-walled pressure-resistant bottle, and analytically pure tetrahydrofuran (20 mL), propylene oxide (4.6 mL, 66 mmol, 10 equivalent) and triphosgene (976 mg, 3.3 mmol, 0.5 equivalent) were added. The reaction was stirred at room temperature for 24 hours, washed with water, dried, crystallized to obtain the final product L-mandelic acid O-carboxyanhydride (575 mg, yield 49%).

The NMR and carbon spectra of L-mandelic acid O-carboxyanhydride are as follows:
**¹H NMR**(400 MHz, DMSO-*d*₆) δ 7.47 - 7.40(m, 2H), 7.39 - 7.24(m, 3H), 5.04(s, 1H).
**¹³C NMR**(101 MHz, DMSO-*d*₆) δ 174.2, 140.3, 128.2, 127.8, 126.7, 72.5.

### Example 19: Synthesis of D-penicillamine N-carboxyanhydride

D-penicillamine (1.0 g, 6.7 mmol, 1 equivalent) was added into a thick-walled pressure-resistant bottle, then 15 mL of analytically pure tetrahydrofuran, propylene oxide (5.8 mL, 82.5 mmol, 10 equivalent), and triphosgene (1.22 g, 4.1 mmol, 0.5 equivalent) were added. The reaction was stirred at room temperature for 1.5 hours, spin-dried, crystallized to obtain the final product D-penicillamine N-carboxyanhydride (771 mg, yield 53%).
The NMR and carbon spectra of D-penicillamine N-carboxyanhydride are as follows:
**¹H NMR**(400 MHz, DMSO-*d*₆) δ 13.18(s, 1H), 8.24(s, 1H), 4.12(d, *J=* 1.0 Hz, 1H), 1.62(s, 3H), 1.41(s, 3H).
**¹³C NMR**(101 MHz, DMSO-*d*₆) δ 171.6, 170.2, 66.5, 52.9, 29.8, 25.9.

### Example 20: Synthesis of O-tert-butyl L-serine N-carboxyanhydride

O-tert-butyl L-serine (1.0 g, 6.2 mmol, 1 equivalent) was added to a thick-walled pressure-resistant bottle, 15 mL of analytically pure tetrahydrofuran, propylene oxide (4.3 mL, 62.0 mmol, 10 equivalent), and triphosgene (930.1 mg, 3.1 mmol, 0.5 equivalent) were added. The reation was stirred for 1.5 hours in total, passed through silica gel comlun to obtain the final product, O-tert-butyl L-serine N-carboxyanhydride (1.0081 g, yield 87%).
The NMR and carbon spectra of O-tert-butyl L-serine N-carboxyanhydride are as follows:
**¹H NMR**(400 MHz, DMSO-*d*₆) δ 8.95(br, 1H), 4.57(dt, *J* = 2.8, 1.5 Hz, 1H), 3.62(dd, *J* = 10.3, 2.8 Hz, 1H), 3.50(dd, *J* = 10.3, 2.8 Hz, 1H), 1.10(s, 9H).
**¹³C NMR**(101 MHz, DMSO-*d*₆) δ 170.2, 152.3, 73.2, 59.9, 58.6, 27.1.

### Example 21: Synthesis of 1-Methyl N-(tert-butoxycarbonyl)-L-aspartate N-carboxyanhydride

**1-Methyl N-(tert-butoxycarbonyl)-L-aspartate** (1.0 g, 4.0 mmol, 1 equivalent) was added into a 50 ml eggplant flask, 15 mL of analytically pure acetonitrile, and propylene oxide (2.8 mL, 40.0 mmol, 10 equivalent) were added under magnetic stirring. After stirring evenly, triphosgene (613.2 mg, 2.0 mmol, 0.5 equivalent) was added under atmospheric conditions to react for two hours at 0°C, dried, and the final product **1-methyl N-(tert-butoxycarbonyl)-L-aspartate** N-carboxyanhydride (500 mg, yield 71%) was obtained.

The NMR and carbon spectra of **1-methyl N-(tert-butoxycarbonyl)-L-aspartate** N-carboxyanhydride are as follows:
**¹H NMR**(400 MHz, DMSO-*d*₆) δ 8.96(d, *J* = 4.4 Hz, 1H), 4.32(ddd, *J* = 7.2, 4.4, 3.0 Hz, 1H), 3.70(s, 3H), 3.23(dd, *J* = 16.8, 7.2 Hz, 1H), 2.91(ddd, *J* = 16.8, 3.0, 1.1 Hz, 1H).
**¹³C NMR**(101 MHz, DMSO-*d*₆) δ 170.9, 165.1, 148.9, 52.9, 48.4, 30.7.

### Example 22: Synthesis of N-ε-(tert-butoxycarbonyl)-L-lysine N-carboxyanhydride

N-ε-(tert-butoxycarbonyl)-L-lysine (1.0 g, 4.1 mmol, 1 equivalent) was added into a thick-walled pressure-resistant bottle, and analytically pure tetrahydrofuran (15 mL) was added, followed by the addition of propylene oxide (2.8 mL, 41.0 mmol, 10 equivalent) under magnetic stirring. After stirring evenly, triphosgene (620.2 mg, 2.0 mmol, 0.5 equivalent) was added under atmospheric conditions and reacted at room temperature for 2 hours, washed with water, dried, crystallized to obtain the final product N-ε-(tert-butoxycarbonyl) -L-lysine N-carboxyanhydride (888 mg, yield 80%).

The NMR and carbon spectra of N-ε-(tert-butoxycarbonyl)-L-lysine N-carboxyanhydride are as follows:
**¹H NMR**(400 MHz, DMSO-*d*₆) δ 9.07(br, 1H), 6.79(t, *J* = 6.0 Hz, 1H), 4.42(dd, *J* = 7.3, 5.2 Hz, 1H), 2.89(q, *J* = 6.0 Hz, 2H), 1.78 - 1.57(m, 2H), 1.37(s, 9H), 1.43 - 1.19(m, 4H).
**¹³C NMR**(101 MHz, DMSO-*d*₆) δ 171.7, 155.6, 152.0, 77.4, 57.0, 30.6, 28.9, 28.3, 21.6.

### Example 23: Synthesis of N-ε-trifluoroacetyl-L-lysine N-carboxyanhydride

N-ε-trifluoroacetyl-L-lysine (1.0 g, 4.1 mmol, 1 equivalent) was added into a thick-walled pressure-resistant bottle, and analytically pure tetrahydrofuran (15 mL) was added, followed by the addition of propylene oxide (2.8 mL, 41.0 mmol, 10 equivalent) under magnetic stirring. After stirring evenly, triphosgene (620.2 mg, 2.0 mmol, 0.5 equivalent) was added under atmospheric conditions. The reaction was carried out at room temperature for 2 hours, washed with water, dried, crystallized to obtain the final product N-ε-trifluoroacetyl-L-lysine N-carboxyanhydride (900 mg, yield 81%).
The NMR spectrum of N-ε-trifluoroacetyl-L-lysine N-carboxyanhydride is as follows:
¹H NMR(400 MHz, DMSO-*d*₆, δ) 9.40(t, 1H), 9.09(s, 1H), 4.43(t, 1H), 3.17(q, 2H), 1.86-1.58(m, 2H), 1.50(p, 2H), 1.44-1.21(m, 2H).

### Example 24: Synthesis of L-glutamic acid N-carboxyanhydride

L-glutamic acid (1.0 g, 6.8 mmol, 1 equivalent) was added into a thick-walled pressure-resistant bottle, 15 mL of analytically pure tetrahydrofuran was added, and epichlorohydrin (2.1 mL, 68.0 mmol, 10 equivalent) was added under magnetic stirring. After stirring evenly, triphosgene (1.01 g, 3.4 mmol, 0.5 equivalent) was added under atmospheric conditions. The reaction was carried out at room temperature for 20 hours, dried, crystallized to obtain the final product L-glutamic acid N-carboxyanhydride (1.0 g, yield 89%).

The NMR and carbon spectra of L-glutamic acid N-carboxyanhydride are as follows:
¹H NMR(400 MHz, DMSO-*d*₆) δ 12.27(br, 1H), 9.09(br, 1H), 4.46(ddd, *J* = 7.5, 5.5, 1.2 Hz, 1H), 2.37(t, *J* = 7.5 Hz, 2H), 2.00(dtd, *J* = 14.0, 7.5, 5.5 Hz, 1H), 1.87(dq, *J* = 14.7, 7.5 Hz, 1H).
¹³C NMR(101 MHz, DMSO-*d*₆) δ 173.4, 171.5, 152.0, 56.3, 29.1, 26.6.

### Example 25: Comparative experiment of absorbents

Taking the synthesis of (*S*)-2-amino-4-((3-(benzylamino)-3-oxypropyl)seleno)butyric acid N-carboxyanhydride as an example, the effects of different absorbents on the separation yield were compared.

(*S*)-2-amino-4-((3-(benzylamino)-3-oxypropyl)seleno)butyric acid hydrochloride (1.0 g, 2.7 mmol, 1 equivalent) was added to a thick-walled pressure-resistant bottle, then 15 mL of analytically pure tetrahydrofuran, propylene oxide (2.0 mL, 27 mmol, 10 equivalent) and triphosgene (397 mg, 1.3 mmol, 0.5 equivalent) were added. The reaction was stirred at room temperature for 1.5 hours, quenched, and passed through a column to obtain the final product (*S*)-2-amino-4-((3-(benzylamino)-3-oxypropyl)seleno)butyric acid N-carboxyanhydride (570 mg, yield 58%).

(*S*)-2-amino-4-((3-(benzylamino)-3-oxypropyl)seleno)butyric acid hydrochloride (1.0 g, 2.7 mmol, 1 equivalent) was added to a reaction bottle, and analytically pure tetrahydrofuran (15 mL), α-pinene (0.6 mL, 3.8 mmol, 1.4) and triphosgene (346 mg, 1.2 mmol, 0.4 equivalent) were added and stirred at 50°C for 5 hours. The amino acid cannot be dissolved, and the corresponding product cannot be obtained by column separation, and the separation yield is 0%. It is speculated that pinene, as an absorbent, will react only a little after long-term heating and stirring. Column separation was tried, but the product will decompose quickly on the column.

### Example 26: Experiment of effects of different epoxy compounds

Taking the synthesis of benzyl L-glutamate N-carboxyanhydride as an example, the specific experimental process is the same as that of Example 1, except that ethylene oxide, dimethylethylene oxide or cyclohexyloxirane are used as absorbents, respectively.

### 1. Ethylene oxide absorbent

Benzyl L-glutamate (1.0 g, 4.2 mmol, 1 equivalent) was added into a thick-walled pressure-resistant bottle, and 9 mL of analytically pure tetrahydrofuran, ethylene oxide in THF (8 mL, 3 M in THF, 6 equivalents), and triphosgene (637.3 mg, 2.1 mmol, 0.5 equivalent) were added. The reaction was stirred at room temperature for 1.5 hours, washed with water, crystallized to obtain the final product benzyl L-glutamate N-carboxyanhydride (1.0 g, yield 93%).

### 2. Gem-dimethylethylene oxide absorbent

Benzyl L-glutamate (1.0 g, 4.2 mmol, 1 equivalent) was added into a thick-walled pressure-resistant bottle, and 15 mL of analytically pure tetrahydrofuran, gem-dimethylethylene oxide (1.47 g, 20 mmol, 4.8 equivalent) and triphosgene (642.5mg, 2.1 mmol, 0.5 equivalent) were added. The reaction was stirred at room temperature for 1.5 hours, spin-dried, passed through a silica gel column to obtain the final product benzyl L-glutamate N-carboxyanhydride (272.2mg, yield 25%).

### 3. Cyclohexyloxirane absorbent

Benzyl L-glutamate (1.0 g, 4.2 mmol, 1 equivalent) was added into a thick-walled pressure-resistant bottle, and 15 mL of analytically pure tetrahydrofuran, cyclohexyloxirane (2.5 g, 25 mmol, 6.0 equivalent) and triphosgene (626.4mg, 2.1 mmol, 0.5 equivalent) were added. The reaction was stirred at room temperature for 1.5 hours, spin-dried, passed through a silica gel column to obtain the final product benzyl L-glutamate N-carboxyanhydride (546.3mg, yield 49%).

### 4. Epichlorohydrin absorbent

Benzyl L-glutamate (1.0 g, 4.2 mmol, 1 equivalent) was added into a thick-walled pressure-resistant bottle, and 15 mL of analytically pure tetrahydrofuran, epichlorohydrin (1.3 mL, 16.8 mmol, 4 equivalents) and triphosgene (632.5 mg, 2.1 mmol, 0.5 equivalent) were added. The reaction was stirred at room temperature for 1.5 hours, washed with water, crystallized, to obtain the final product benzyl L-glutamate N-carboxyanhydride (959 mg, yield 86%).

The experimental results are summarized in Table 1.

**Table 1: Effect of epichlorohydrin absorbent**

| | 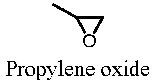 | 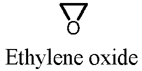 | 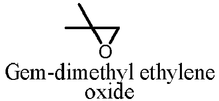 | 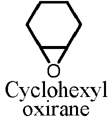 | 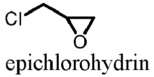 |
|---|---|---|---|---|---|
| Purification method | Crystallizaion | Crystallizaion | Column chromatography | Column chromatography | Crystallizaion |
| Yield | 93% | 93% | 25% | 49% | 86% |
| Equivalent | 4 - 10 | 5 | 10 | 4 - 6 | 4 |

### Example 27: Synthesis of β-alanine N-carboxyanhydride

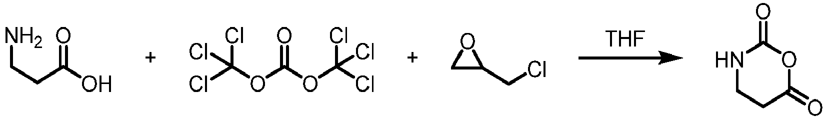

β-alanine (1.0 g, 11.2 mmol, 1 equivalent) was added into a thick-walled pressure-resistant bottle, 40 mL of analytically pure tetrahydrofuran was added, and epichlorohydrin (3.5 mL, 44.9 mmol, 4 equivalents) was added under magnetic stirring. After stirring evenly, triphosgene (1.67 g, 5.6 mmol, 0.5 equivalent) was added under atmospheric conditions. The reaction was carried out at room temperature for 14 hours, spin-dried, crystallized to obtain the final product β-alanine N-carboxyanhydride (879 mg, yield 68%).

The NMR and carbon spectra of β-alanine N-carboxyanhydride are as follows:
¹H NMR(400 MHz, DMSO-*d*₆) δ 8.38(s, 1H), 3.26(td, *J* = 6.7, 2.8 Hz, 2H), 2.75(t, *J* = 6.7 Hz, 2H).
¹³C NMR(101 MHz, DMSO-*d*₆) δ 167.0, 149.6, 34.3, 28.3.

### Example 28: Synthesis of 3-amino-3-(4-methylphenyl)propionic acid N-carboxyanhydride

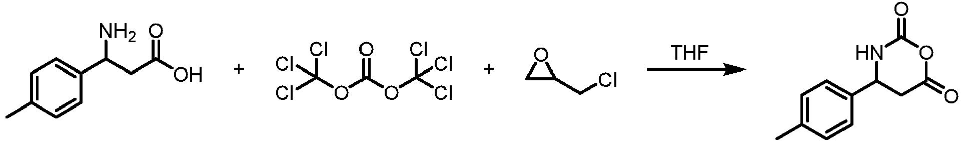

3-Amino-3-(4-methylphenyl)propionic acid (1.0 g, 5.6 mmol, 1 equivalent) was added into a thick-walled pressure-resistant bottle, 30 mL of analytically pure tetrahydrofuran was added, and epichlorohydrin (1.8 mL, 22.3 mmol, 4 equivalents) was added under magnetic stirring. After stirring evenly, triphosgene (826.6 mg, 2.8 mmol, 0.5 equivalent) was added under atmospheric conditions. The reaction was carried out at room temperature for 4 hours, spin-dried, crystallized to obtain the final product of 3-amino-3-(4-methylphenyl)propionic acid N-carboxyanhydride (1.017 g, yield 89%).

The NMR and carbon spectra of 3-amino-3-(4-methylphenyl)propionic acid N-carboxyanhydride are as follows:
¹H NMR(400 MHz, DMSO-*d*₆) δ 8.90(d, *J* = 3.0 Hz, 1H), 7.21(s, 4H), 4.76(td, *J* = 6.2, 3.0 Hz, 1H), 3.13(dd, *J* = 16.1, 6.2 Hz, 1H), 2.94(dd, *J* = 16.1, 6.2 Hz, 1H), 2.29(s, 3H).
¹³C NMR(101 MHz, DMSO) δ 166.1, 149.3, 137.5, 136.7, 129.4, 126.0, 49.0, 36.3, 20.7.

### Example 29: Synthesis of 3-amino-3-(4-chlorophenyl)propionic acid N-carboxyanhydride

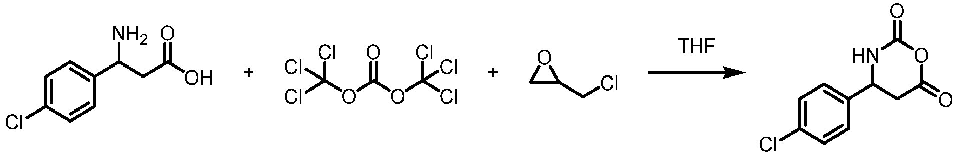

3-Amino-3-(4- chlorophenyl)propionic acid (1.0 g, 5.0 mmol, 1 equivalent) was added into a thick-walled pressure-resistant bottle, 30 mL of analytically pure tetrahydrofuran was added, and epichlorohydrin (1.6 mL, 20.0 mmol, 4 equivalents) was added under magnetic stirring. After stirring evenly, triphosgene (755.6 mg, 2.5 mmol, 0.5 equivalent) was added under atmospheric conditions. The reaction was carried out at room temperature for 3 hours, spin-dried, crystallized to obtain the final product 3-amino-3-(4-chlorophenyl)propionic acid N-carboxyanhydride (953 mg, yield 84%).

The NMR and carbon spectra of 3-amino-3-(4-chlorophenyl)propionic acid N-carboxyanhydride are as follows:
¹H NMR(400 MHz, DMSO-*d*₆) δ 8.95(d, *J* = 2.8 Hz, 1H), 7.48(d, *J* = 8.1 Hz, 2H), 7.37(d, *J* = 8.1 Hz, 2H), 4.86 - 4.80(m, 1H), 3.15(dd, *J* = 16.1, 5.5 Hz, 1H), 2.98(dd, *J* = 16.1, 7.2 Hz, 1H).
¹³C NMR(101 MHz, DMSO) δ 165.8, 149.2, 138.6, 132.8, 128.8, 128.2,48.8, 36.0.

### Example 30: Synthesis of ε-N-trifluoroacetyl L-lysine N-carboxyanhydride

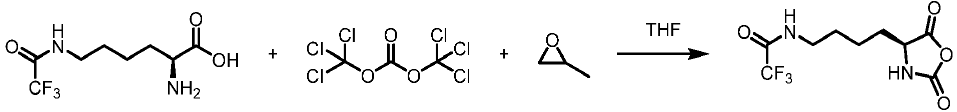

ε-N-trifluoroacetyl L- lysine (1.0 g, 4.1 mmol, 1 equivalent) was added into a thick-walled pressure-resistant bottle, analytically pure tetrahydrofuran (20 mL) was added, and propylene oxide (1.2 mL, 16.4 mmol, 4 equivalent) was added under magnetic stirring. After stirring evenly, triphosgene (624.3 mg, 2.0 mmol, 0.5 equivalent) was added under atmospheric conditions. The reaction was carried out at room temperature for 3 hours, spin-dried, crystallized to obtain the final product ε-N-trifluoroacetyl L-lysine N-carboxyanhydride (953 mg, yield 84%).

The NMR and carbon spectra of ε-N-trifluoroacetyl L-lysine N-carboxyanhydride are as follows:
¹H NMR(400 MHz, DMSO-*d*₆) δ 9.42(t, *J* = 6.5 Hz, 1H), 9.10(s, 1H), 4.43(t, *J* = 6.5 Hz, 1H), 3.17(q, *J*= 6.5 Hz, 2H), 1.75(ddt, *J* = 15.5, 10.6, 5.2 Hz, 1H), 1.69 - 1.59(m, 1H), 1.49(p, *J* = 7.4 Hz, 2H), 1.43 - 1.34(m, 1H), 1.34 - 1.21(m, 1H).
¹³C NMR(101 MHz, DMSO) δ 171.7, 156.8, 156.4, 156.0, 155.7, 152.0, 120.3, 117.4, 114.6, 111.7, 57.0, 30.6, 27.7, 21.6.

### Example 31: Synthesis of L-valine N-carboxyanhydride

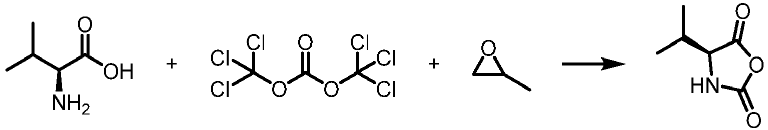

L-valine (1.0 g, 8.5 mmol, 1 equivalent) was added into a thick-walled pressure-resistant bottle, 20 mL of analytically pure tetrahydrofuran was added, and propylene oxide (2.4 mL, 34.0 mmol, 4 equivalents) was added under magnetic stirring. After stirring evenly, triphosgene (1.29 g, 4.3 mmol, 0.5 equivalent) was added under atmospheric conditions. The reaction was carried out at room temperature for 4 hours, washed with water, dried, crystallized to obtain the final product L-valine N-carboxyanhydride (890 mg, yield 72%).

The NMR and carbon spectra of L-valine N-carboxyanhydride are as follows: ¹H NMR(400 MHz, Chloroform-*d*) δ 6.85(br, 1H), 4.22(dd, *J* = 4.2, 1.0 Hz, 1H), 2.25(heptd, *J* = 6.9, 4.2 Hz, 1H), 1.08(d, *J* = 6.9 Hz, 3H), 1.03(d, *J* = 6.9 Hz, 3H). ¹³C NMR(101 MHz, Chloroform-*d*) δ 169.1, 153.7, 63.2, 30.9, 18.3, 16.7.

### Example 32: Synthesis of L-leucine N-carboxyanhydride

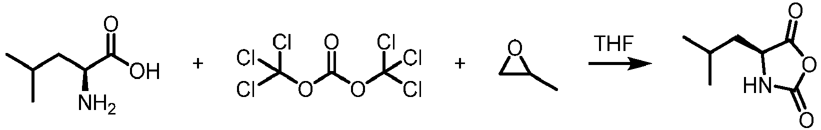

L-leucine (2.0 g, 15.2 mmol, 1 equivalent) was added into a thick-walled pressure-resistant bottle, 40 mL of analytically pure tetrahydrofuran was added, and propylene oxide (6.0 mL, 91.0 mmol, 6 equivalents) was added under magnetic stirring. After stirring evenly, triphosgene (2.30 g, 7.6 mmol, 0.5 equivalent) was added under atmospheric conditions. The reaction was carried out at room temperature for 3 hours, washed with water, dried, crystallized to obtain the final product L-leucine N-carboxyanhydride (1.70g, yield 70%).

The NMR and carbon spectra of L-leucine N-carboxyanhydride are as follows:
¹H NMR(400 MHz, DMSO-d6) δ 9.13(br, 1H), 4.45(dd, J = 8.7, 5.5 Hz, 1H), 1.81 - 1.65(m, 1H), 1.65 - 1.49(m, 2H), 0.92 - 0.85(m, 6H).
¹³C NMR(101 MHz, DMSO-*d*₆) δ 172.1, 152.0, 55.6, 40.1, 24.2, 22.8, 21.2.

### Example 33: Synthesis of poly β-alanine N-carboxyanhydride

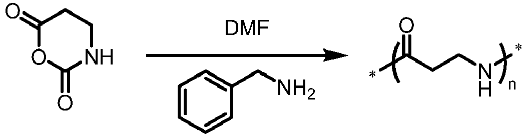

β-alanine N-carboxyanhydride (150 mg, 1.3 mmol, 50 equivalents) was added into a glass bottle, 4 mL of chromatographic pure N,N- dimethylformamide was added, and 26 microliters of benzylamine initiator (1 M in N,N-dimethylformamide) was added. The reaction was stirred at room temperature for 30 hours, precipitated with ethylether and dried in vacuum to obtain the final product poly β-alanine N-carboxyanhydride (91 mg, yield 99%). Polymerization degree: 50.

The NMR spectrum of poly β-alanine N-carboxyanhydride is as follows:
1H NMR(400 MHz, Chloroform-d) δ 3.78 - 3.62(m, 2H), 2.86 - 2.72(m, 2H).

### Example 34: Comparative experiment of the synthesis of poly L-glutamic acid N-carboxyanhydride

### 1. Previous synthetic route

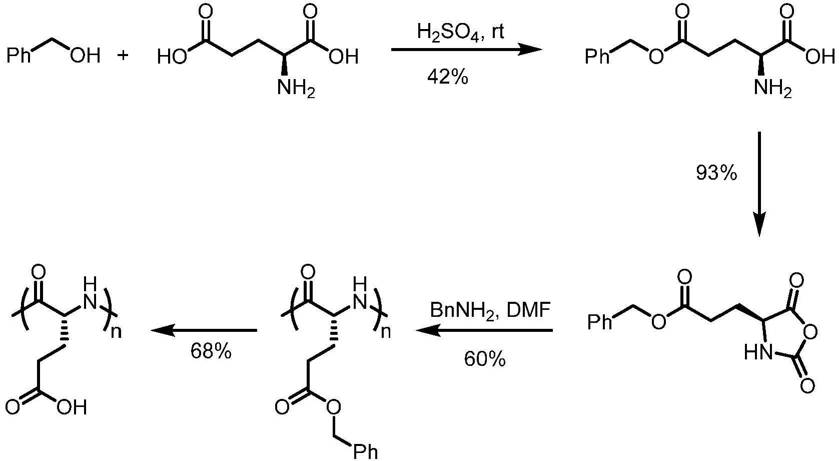

Into a conical flask, 10 g L-glutamic acid and 50 mL benzyl alcohol were weighed, 6 mL concentrated sulfuric acid was added dropwise at 0°C and reacted at 0°C for 16 h. The reaction was neutralized and washed with isopropanol and triethylamine, precipitated by ethylether and dried to obtain 10g of benzyl L-glutamate, yield 42%.

Benzyl L-glutamate (5.0 g, 21.1 mmol, 1 equivalent) was added into a thick-walled pressure-resistant bottle, and 60 mL of analytically pure tetrahydrofuran was added. Under magnetic stirring, propylene oxide (15.3 mL, 211 mmol, 10 equivalents) was added. After stirring evenly, triphosgene (3.184 g, 10.5 mmol, 0.5 equivalents) was added under atmospheric conditions. The reaction was stirred at room temperature for 1.5 hours, washed with water, extracted with ethyl acetate, dried, and subjected to diffusion crystallization to obtain the final product benzyl L-glutamate N-carboxyanhydride (5.159 g, yield 93%).

Benzyl L-glutamate N-carboxyanhydride (201 Mg, 0.8 mmol, 50 equivalents) was added into a glass bottle, 4 mL of chromatographic pure N,N-dimethylformamide was added, and 15 microliters of benzylamine initiator (1 M in N,N-dimethylformamide) was added. The reaction was carried out at room temperature for 30 hours, precipitated with ethylether and dried in vacuum to obtain the final product Poly L-glutamic acid N-carboxyanhydride (100 mg, yield 60%).

Poly L-glutamic acid N-carboxyanhydride (100 mg) was dissolved in 5 mL of tetrahydrofuran, 1 mL of hydrobromic acid in acetic acid was added, the reaction was carried out at room temperature under sealing for 3 days, and precipitated to obtain 40 mg of poly L-glutamic acid, yield 68%.

Yield of four steps in total is: 16%.

### 2. Direct two-step synthesis of poly L-glutamic acid N-carboxyanhydride

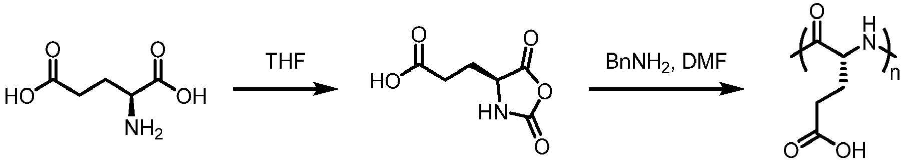

L-glutamic acid (1.0 g, 6.8 mmol, 1 equivalent) was added into a thick-walled pressure-resistant bottle, 15 mL of analytically pure tetrahydrofuran was added, and epichlorohydrin (2.1 mL, 68.0 mmol, 10 equivalents) was added under magnetic stirring. After stirring evenly, triphosgene (1.01 g, 3.4 mmol, 0.5 equivalents) was added under atmospheric conditions. The reaction was carried out at room temperature for 20 hours, dried, crystallized to obtain the final product L-glutamic acid N-carboxyanhydride (1.0 g, yield 89%).

L-glutamic acid N-carboxyanhydride (200 mg, 1.2 mmol, 20 equivalents) was added into a glass bottle, 4 mL of chromatographic pure N,N-dimethylformamide was added, and 58 microliters of benzylamine initiator (1 M in N,N-dimethylformamide) was added. The reaction was carried out at room temperature for 30 hours, precipitated with ethylether and dried in vacuum to obtain the final product poly L-glutamic acid N-carboxyanhydride (120 mg, yield 80%). The polymerization degree N is 20.

The yield of two steps in total is: 72%.

The NMR spectrum of poly L-glutamic acid N-carboxyanhydride is as follows:
**¹H NMR**(400 MHz, DMSO-*d*₆) δ 4.40 - 3.92(m, 1H), 3.89 - 3.11(m, 2H), 2.41 - 2.16(m, 1H), 2.04 - 1.67(m, 1H).
SEC characterization is shown in Figure 2.

It should be understood that although the present invention has been described in conjunction with its detailed description, the foregoing description is intended to illustrate rather than limit the scope of the present invention as defined by the scope of the appended claims. Other aspects, advantages and modifications are within the scope of the appended claims.

The corresponding structural formulas and names of different amino acids and products in the examples are as follows:

### Amino acids:

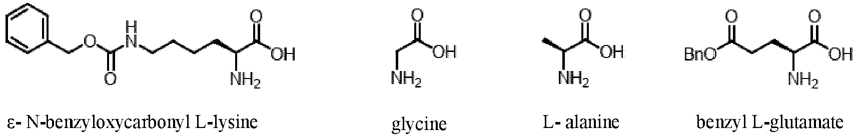

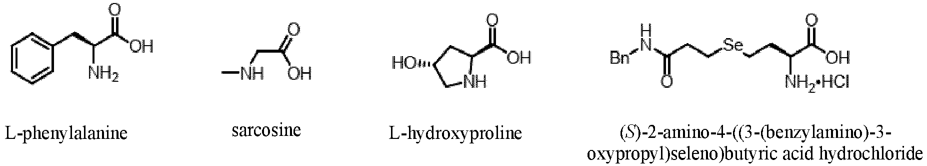

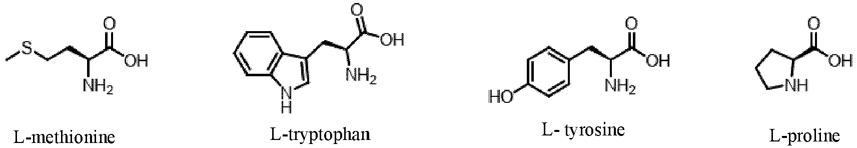

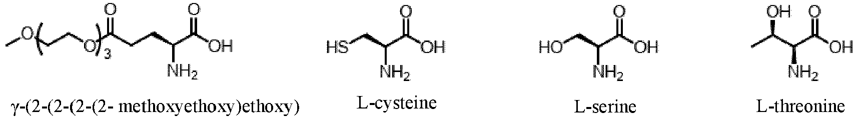

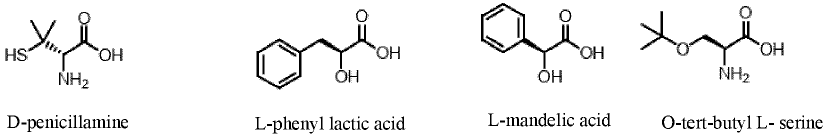

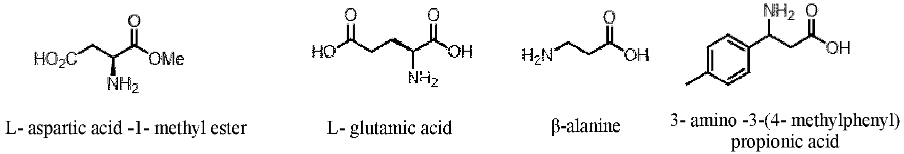

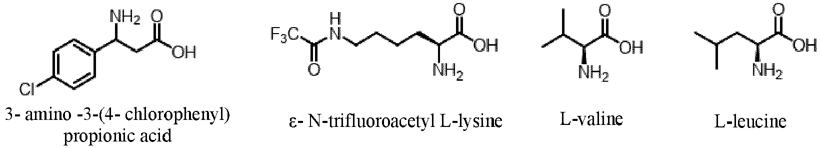

### Products

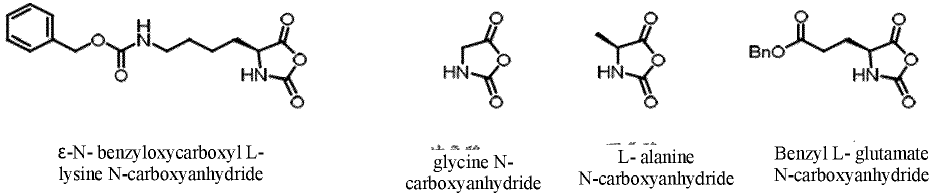

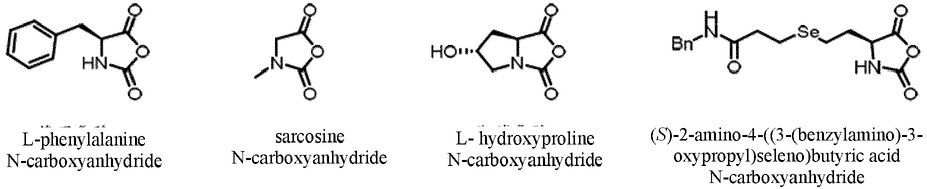

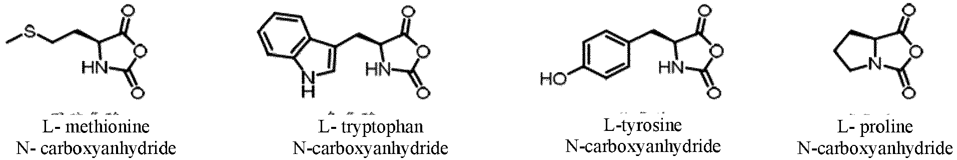

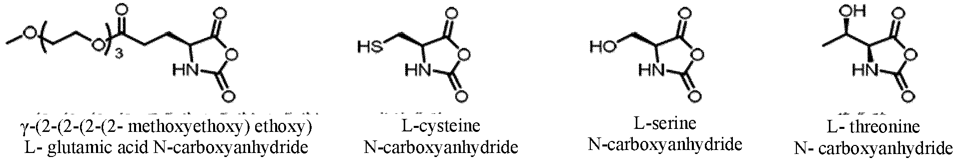

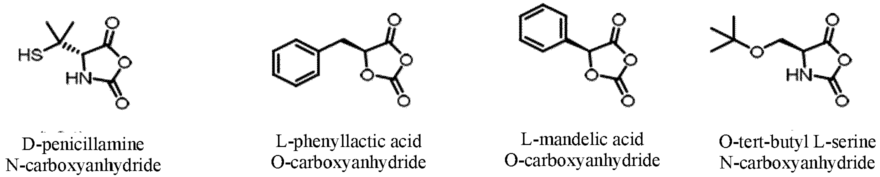

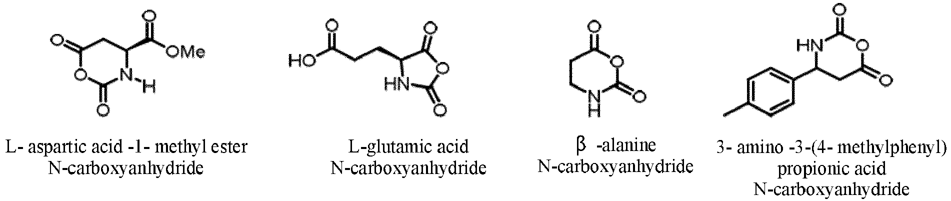

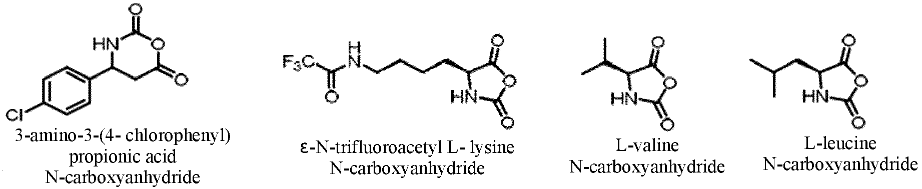

## Claims

1. A method for preparing a compound of formula (II), which comprises the steps of reacting a compound of formula (I) with a cyclizing agent to produce a compound of formula (II) and an acid, and using an epoxy compound as an acid removal agent, 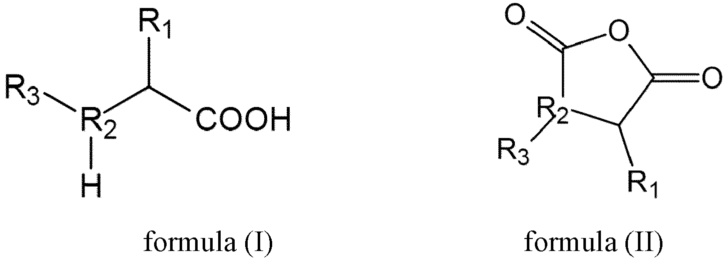 wherein:
R₁ is -R₄-R₅-R₆, wherein R₄ is H, optionally substituted C₁₋₆ linear or branched alkyl (for example, C₁₋₆ linear or branched alkyl substituted by hydroxyl or sulfhydryl), optionally substituted C₁₋₆ alkoxy, carboxyl, amino, carbonylamino or aminocarbonyl, guanidyl, optionally substituted phenyl (for example, substituted by hydroxyl), optionally substituted benzyl (for example, substituted by hydroxyl), indolyl, imidazolyl, guanidyl or carbonylalkoxy or alkoxycarbonyl;
R₅ is absent, oxy, seleno, thio, carbonyl, ester group, ester group imino or imino ester group, imino, amino, carbonylamino, carbonylimino or iminocarbonyl, benzyl ester, optionally substituted phenyl (for example, substituted by hydroxyl), optionally substituted benzyl (for example, substituted by hydroxyl), indolyl, imidazolyl, guanidyl, carbonylalkoxy or alkoxycarbonyl, hydroxyl, sulfhydryl, or carboxyl;
R₆ is absent, H, optionally substituted C₁₋₆ linear or branched alkyl (for example, C₁₋₆ linear or branched alkyl substituted by hydroxyl, sulfhydryl or halogen); optionally substituted C₁₋₆ alkoxy, hydroxyl, carboxyl, sulfhydryl, amino, carbonylamino, guanidyl, optionally substituted phenyl (for example, substituted by hydroxyl), amino protecting group, hydroxyl protecting group or carboxyl protecting group, optionally substituted benzyl (for example, substituted by hydroxyl), optionally substituted phenylalkyl, optionally substituted benzylalkyl, indolyl, imidazolyl, guanidyl, carbonylalkoxy or alkoxycarbonyl, benzyl ester group, benzyliminocarbonylalkyl, trifluoroacetyl, tert-butoxycarbonyl or - [O(CH₂)ₘ₁]ₘ₂-O-R₇,
wherein R₇ is H, optionally substituted C₁₋₆ linear or branched alkyl (for example, C₁₋₆ linear or branched alkyl substituted by hydroxyl or sulfhydryl), optionally substituted C₁₋₆ alkoxy, carbonylalkyl, or iminoalkyl; m1 and m2 are each independently integers from 1 to 6;
R₂ is N, O or S, or R₁ and R₂ are taken together with the carbon atoms to which they are attached to form a 3- to 7-membered ring, the 3- to 7-membered ring is optionally substituted by halogen, sulfhydryl or hydroxyl, the 3- to 7-membered ring is, for example, indolyl, imidazolyl, pyrrolidinyl, sulfhydrylpyrrolidinyl or hydroxypyrrolidinyl; when R₂ is O or S, H connected to R₂ is optionally substituted by hydroxyl protecting group or sulfhydryl protecting group;
R₃ is absent, H, halogen, optionally substituted C₁₋₆ linear or branched alkyl (for example, C₁₋₆ linear or branched alkyl substituted by hydroxyl or sulfhydryl), optionally substituted C₂₋₆ linear or branched alkenyl or alkynyl, C₁₋₆ alkoxy, cycloalkyl, aryl or heterocyclyl, wherein one or more hydrogen atoms are optionally substituted by halogen, oxygen or nitrogen, or amino protecting groups, such as Boc or Cbz.

2. The preparation method according to claim 1, wherein the epoxy compound as the acid removal agent has the structure of formula (III): 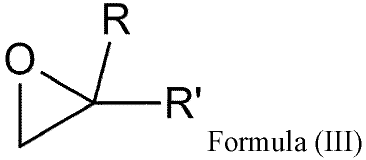 wherein: R and R' are each independently H, halogen, optionally substituted C₁₋₆ linear or branched alkyl, optionally substituted C₂₋₆ linear or branched alkenyl or alkynyl, alkoxy or cycloalkyl, wherein one or more hydrogen atoms are optionally substituted by halogen, C₁₋₆ linear or branched alkyl, oxygen or nitrogen; or one of R and R' together with the two carbon atoms in the epoxy group to form a five-to seven-membered ring.

3. The preparation method according to claim 1 or 2, wherein the epoxy compound is selected from one or more of the following: ethylene oxide, propylene oxide, 1,2-butylene oxide, dimethylethylene oxide, cyclohexene oxide and halogen-substituted derivatives thereof, such as epichlorohydrin.

4. The preparation method according to any one of claims 1-3, wherein the cyclizing agent is selected from one or more of the following: phosgene, diphosgene, triphosgene, phosphorus trichloride and phosphorus pentachloride.

5. The preparation method according to any one of claims 1 to 4, wherein R₁ is H, optionally substituted C₁₋₆ linear or branched alkyl, optionally substituted aryl or BnNHCOCH₂CH₂SeCH₂CH₂; preferably, the optionally substituted C₁₋₆ linear or branched alkyl is linear or branched C₁₋₆ alkyl, C₁₋₆ alkylhydroxyl, C₁₋₆ alkylsulfhydryl, C₁₋₆ alkylcarboxyl, C₁₋₆ alkylamino, C₁₋₆ alkylcarbonylamino, C₁₋₆ alkylaryl, C₁₋₆ alkylheterocyclyl, C₁₋₆ alkylguanidyl, C₁₋₆alkylthioC₁₋₆ alkyl, C₁₋₆ alkoxyC₁₋₆ alkyl; preferably wherein the aryl or heterocyclyl is optionally substituted, for example, substituted by hydroxyl or sulfhydryl; preferably, wherein the amino group, hydroxyl group or carboxyl group is protected;
preferably, wherein R₁ is H, CH₃, CH(CH₃)₂, CH₂CH(CH₃)₂, CH(CH₃)CH₂CH₃, CH₂-C₆H₅, methylindolyl, CH₂-C₆H₄-OH, CH₂-COOH, CH₂-CONH₂, (CH₂)₂-COOH, (CH₂)₄-NH₂, (CH₂)₂-CONH₂, (CH₂)₂-S-CH₃, CH₂-OH, CH(CH₃)-OH, CH₂-SH, methylimidazolyl, BnCO₂NH(CH₂)₄, BnCO₂(CH₂)₂, CH₂-CH₂-CH₂-CN₃H₄, BnNHCOCH₂CH₂SeCH₂CH₂, CH₃O(CH₂CH₂O)₃COCH₂CH₂, SHC(CH₃)₂, C₆H₅, C(CH₃)₃OCH₃ or trifluoroacetyliminobutyl.

6. The preparation method according to any one of claims 1-5, wherein R₂ is N or O, preferably wherein R₃ is absent, H or methyl, or wherein R₁ and R₂ are taken together with the carbon atoms to which they are attached to form pyrrolidinyl or hydroxypyrrolidinyl, and optionally the imino group is protected.

7. The preparation method according to any one of claims 1 to 6, wherein the compound of formula (I) is an amino acid or a derivative thereof, such as an amino acid protected by Boc or Cbz, and is selected from the following: glycine, alanine, valine, leucine, isoleucine, methionine, proline, tryptophan, serine, tyrosine, cysteine, phenylalanine, asparagine, glutamine, threonine, aspartic acid, glutamic acid, lysine, arginine, histidine, ε-N-benzyloxycarbonyllysine, benzyl glutamate, sarcosine, hydroxyproline, (S)-2-amino-4-((3-(benzylamino)-3-oxypropyl)seleno)butanoic acid, γ-(2-(2-(2-(2-methoxyethoxy)ethoxy)glutamic acid, penicillamine, L-phenyllactic acid, L-mandelic acid, or O-tert-butyl-serine or ε-N-trifluoroacetyl L-lysine, and the compound of formula (II) is the carboxyanhydride of the corresponding amino acid or the derivative thereof, such as N- or O- carboxyanhydride.

8. The preparation method according to any one of claims 1 to 7, wherein the method is carried out under atmospheric conditions and/or at room temperature or without heating.

9. The preparation method according to any one of claims 1-8, further comprising the step of crystallization and/or column chromatography.

10. A method for forming a copolymer, which comprises the steps in the preparation method of any one of claims 1 to 9, and further comprises the step of forming a copolymer from the compound of formula (II), preferably, the copolymer is polyamino acid N-carboxyanhydride.

11. The method of claim 10, wherein R₁ is a carboxyl-containing group, such as COOHCH₂CH₂-, for example, the compound of formula (I) is glutamic acid, and preferably the copolymer is poly L-glutamic acid N-carboxyanhydride.

12. A method for preparing a compound of formula (V), which comprises the steps of reacting a compound of formula (IV) with a cyclizing agent to produce a compound of formula (V) and an acid, and using an epoxy compound as an acid removal agent, 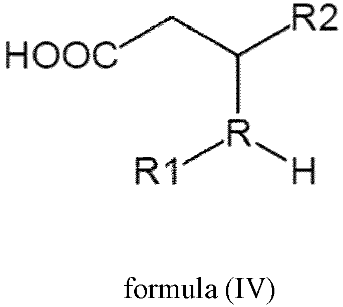 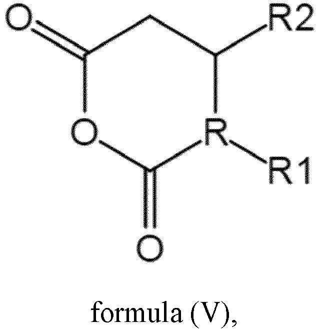 wherein R is N, O or S; R₁ is absent, H, halogen, optionally substituted C₁₋₆ linear or branched alkyl, optionally substituted C₂₋₆ linear or branched alkenyl or alkynyl, C₁₋₆ alkoxy, cycloalkyl, aryl or heterocyclyl, such as benzyl, wherein one or more hydrogen atoms are optionally substituted by halogen, oxygen or nitrogen, or Boc or Cbz; R₂ is H, optionally substituted C₁₋₆ linear or branched alkyl (for example, C₁₋₆ linear or branched alkyl substituted by hydroxyl or sulfhydryl) or optionally substituted C₁₋₆ alkoxy or optionally substituted aryl, such as phenyl substituted by halogen, C₁₋₆ linear or branched alkyl, hydroxyl or sulfhydryl, such as methylphenyl, or carbonyloxyalkyl, such as methyl ester group, ethyl ester group or propyl ester group; when R is O or S, H connected to R is optionally substituted by hydroxyl protecting group or sulfhydryl protecting group.

13. The preparation method according to claim 12, wherein the epoxy compound as the acid removal agent has the structure of formula (III): 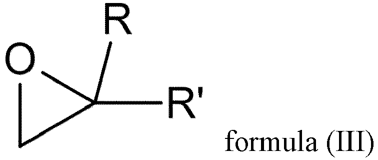 wherein: R and R' are each independently H, halogen, optionally substituted C₁₋₆ linear or branched alkyl, optionally substituted C₂₋₆ linear or branched alkenyl or alkynyl, alkoxy or cycloalkyl; wherein one or more hydrogen atoms are optionally substituted by halogen, C₁₋₆ linear or branched alkyl, oxygen or nitrogen; or one of R and R' together with the two carbon atoms in the epoxy group to form a five- to seven- membered ring.

14. The preparation method according to claim 12, wherein the epoxy compound is selected from one or more of the following: ethylene oxide, propylene oxide, 1,2-butylene oxide, dimethyl ethylene oxide, cyclohexene oxide and halogen-substituted derivatives thereof, such as epichlorohydrin;
preferably, wherein the cyclizing agent is selected from one or more of the following: phosgene, diphosgene, triphosgene, phosphorus trichloride and phosphorus pentachloride;
preferably, wherein the method is carried out under atmospheric condition and/or at room temperature or without heating;
preferably, the preparation method further comprises the step of crystallization and/or column chromatography.

15. The method of claim 13 or 14, wherein the compound of formula (IV) is 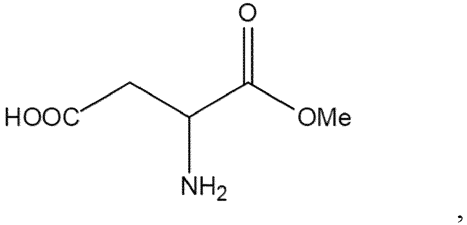 β-alanine, 3-amino-3-(4-methylphenyl)propionic acid or 3-amino-3-(4-chlorophenyl)propionic acid, and the compound of formula (V) is 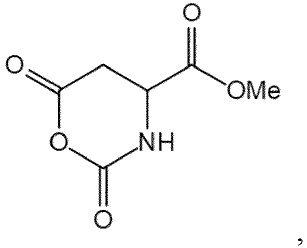 β-alanine N-carboxyanhydride, 3-amino-3-(4-chlorophenyl)propionic acid N-carboxyanhydride or 3-amino-3-(4-methylphenyl)propionic acid N-carboxyanhydride.

16. A method for forming a copolymer, which comprises the steps of the preparation method of any one of claims 12 to 15, and further comprises the step of forming a copolymer from the compound of formula (V).

17. A method for synthesizing a polymer, which comprises preparing a compound of formula (II) by using the method of any one of claims 1 to 9 and/or preparing a compound of formula (V) by using the method of any one of claims 12 to 15, and polymerizing one or more identical or different compounds of formula (II) and/or one or more identical or different compounds of formula (V) to form a polymer, preferably, the polymer is polyamino acid.

18. A method of preparing L-alanine-L-glutamic acid-L-lysine-L-tyrosine polypeptide polymer, which comprises reacting L-alanine, L- glutamic acid derivative, L-lysine derivative or L-tyrosine with cyclizing agent to produce the corresponding amino acid N-carboxyanhydride and an acid, wherein an epoxy compound is used as an acid removal agent in the preparation process of one or more corresponding amino acid N-carboxyanhydrides; then the corresponding amino acid N-carboxyanhydride is polymerized to form L-alanine-L-glutamic acid-L-lysine-L-tyrosine polypeptide polymer, preferably, wherein the cyclizing agent is selected from one or more of the following: phosgene, diphosgene, triphosgene, phosphorus trichloride and phosphorus pentachloride; preferably, the epoxy compound is the epoxy compound defined in claim 2 or 3; preferably, the L-glutamic acid derivative is benzyl L-glutamate, and/or the L-lysine derivative is N-ε-trifluoroacetyl-L-lysine.
